# FASCICULE DE BREVET EUROPEEN

(11) **EP 2 931 294 B1**
(45) Date de publication et mention de la délivrance du brevet: **30.11.2016**
(21) Numéro de dépôt: 13803032.5
(22) Date de dépôt: 12.12.2013
(51) Int. Cl.: A61K 35/36, A61K 35/54, A61L 15/40, A61P 17/02, A61L 15/32

(54) **PANSEMENT CONTENANT DES FIBROBLASTES ET DES KÉRATINOCYTES FOETAUX**
VERBAND MIT FÖTALEN FIBROBLASTEN UND KERATINOZYTEN
BANDAGE CONTAINING FOETAL FIBROBLASTS AND KERATINOCYTES

(30) Priorité: 12.12.2012 FR 1261953
(43) Date de publication de la demande: 21.10.2015
(73) Titulaire: Université de Nantes, 44000 Nantes (FR); CHU Nantes, 44000 Nantes (FR)
(72) Inventeur: DRENO, Brigitte, F-44000 Nantes (FR); ZULIANI, Thomas, F-44000 Nantes (FR); SAIAGH, Soraya, F-44640 Le Pellerin (FR)
(74) Mandataire: Regimbeau
(86) Numéro de dépôt international: PCT/EP2013/076438
(87) Numéro de publication internationale: WO 2014/090961

(56) Documents cités:
- WO-A1-96/33750
- WO-A1-03/068287
- WO-A1-2012/038923
- US-A1- 2002 048 563

## Description

### Art antérieur

La cicatrisation d'une plaie est un phénomène naturel et spontané qui se produit sans l'intervention du chirurgien. Bien que les processus fondamentaux de la coagulation du sang, du dépôt de la fibrine, de la synthèse et de l'organisation du collagène aient été étudiés pendant de nombreuses années, les facteurs qui les déclenchent et les contrôlent sont encore imparfaitement compris. L'évolution de la cicatrisation des plaies peut être modifiée par des manipulations endocrines, pharmacologiques et physiques sophistiquées ou, plus simplement, par un acte chirurgical, mais les effets les plus importants influençant la cicatrisation d'une plaie ouverte résident dans la nature du pansement qui lui est appliqué.

Pour faciliter la cicatrisation d'une plaie, il est possible d'utiliser un pansement gras, un pansement semi-occlusif ou un pansement occlusif (Begaud B. Ann Dermatol Venereol 2002). Cependant, lorsque le déficit cutané est de grande taille ou qu'il reste réfractaire aux traitements conventionnels, des greffes de peau sont parfois nécessaires pour stimuler la cicatrisation. Ces greffes de peau peuvent être prélevées sur le patient lui-même en zone non lésée (autogreffes), mais l'autogreffe demeure parfois difficile car la prise d'un greffon autologue de trop grande taille pose de nouveaux problèmes de cicatrisation au niveau du site donneur, les déficits cutanés de type ulcères veineux touchent des personnes présentant déjà des troubles de la cicatrisation (personnes âgées, diabétiques).

Dans le cas où l'autogreffe n'est pas possible, divers produits de peau artificielle peuvent servir de substituts à la peau naturelle dans le traitement des déficits cutanés et permettre à une peau normale de se reformer (Ho C, et al, Rapport technologique n°52. 2005). Ces substituts peuvent être classés en trois catégories : les greffes d'épiderme cultivé (qui remplacent seulement l'épiderme de surface), les substituts dermiques (qui remplacent la couche dermique inférieure) et les greffes composites (qui consistent en l'association d'une composante dermique et d'une composante épidermique). Plusieurs produits sont disponibles dans chacune de ces catégories. Enfin, une quatrième catégorie est représentée par les kératinocytes en suspension dans une matrice biologique. Toutes les études cliniques antérieures réalisées avec ces différents modèles de substituts cutanés n'ont montré aucun effet indésirable grave. Toutes les applications de ces modèles ont été bien tolérées par les patients.

Les greffons d'épiderme sont généralement obtenus selon la technique de culture de kératinocytes développée en 1975 par Rheinwald et Green (Cell 1975: 6: 331-344). Cette technique permet de produire des feuillets épidermiques en grande quantité, en cultivant des kératinocytes d'un patient ou d'un donneur sur une couche de cellules nourricières (fibroblastes murins irradiés). Les feuillets confluents et différenciés sont transférés sur un support avant la greffe. Les kératinocytes mis en culture peuvent être autologues (pour Laserskin®, Epibase®) ou allogéniques (pour CryoCeal). Le recours à des couches de kératinocytes allogéniques produites grâce à un donneur autre que le patient permet en effet de réduire la période d'attente, mais le risque de transmission de maladies virales est important. L'utilisation de ces épithéliums de culture présente d'autres inconvénients: d'une part, la taille des feuillets diminue de 10 à 50 % lorsqu'ils sont enzymatiquement détachés du support ayant servi à leur culture, et d'autre part, ces feuillets sont fragiles et difficiles à manipuler (Chester DL, et al. J Burn Care Rehabil 2004; Shen JT et Falanga V. J Cutan Med Surg 2003). D'autre part, le taux de prise des greffes est également variable : la greffe n'est souvent stable qu'à court-terme et le néoderme ne se renouvelle que très lentement. Enfin, l'absence de composants dermiques contribue à la contraction de la plaie, à l'instabilité de la greffe et à la formation de bulles au niveau de la greffe.

Pour pallier ces problèmes, des substituts de peau contenant une matrice dermique (par exemple de la marque Apligraf ou Dermagraft) ont été mis au point. Ces substituts n'utilisent que des cellules allogéniques, contrairement aux substituts épidermiques.

Plus précisément, le Dermagraft^{®} est une structure dermique vivante et active du point de vue métabolique, contenant des fibroblastes allogéniques de prépuce de nouveaux-nés cultivés dans un treillis bio-résorbable de polyglactine ou d'acide glycolique (voir US 5,460,939). Ces fibroblastes favorisent la synthèse de facteurs de croissance (PDGF, VEGF, ...), de glycoprotéines et de protéines (collagène, fibronectine, ...) *in situ,* sur la plaie à cicatriser. Cette matrice dermique évite les problèmes de contraction de la plaie et d'instabilité de la greffe (Shen JT et Falanga V. J Cutan Med Surg 2003). Un autre substitut dermique est l'Apligraf^{®} (ou Graftskin^{®}, voir US 4,837,379), qui contient, dans un gel de collagène bovin de type I, des fibroblastes allogéniques de prépuce de nouveaux-nés. Sur cette couche de fibroblastes sont cultivés des kératinocytes allogéniques qui prolifèrent et se différencient. Les cellules qui constituent Apligraf^{®} sont capables de produire leur propre matrice et leurs propres cytokines. Ce substitut ne contient pas de cellules présentatrices d'antigènes telles que les cellules de Langerhans, ou les cellules dendritiques du derme, ni de cellules endothéliales ou de leucocytes. Néanmoins, les essais cliniques visant à démontrer l'efficacité clinique d'Apligraf^{®} ou de Dermagraft^{®} pour le traitement des déficits cutanés mettent insuffisamment en évidence l'effet bénéfique de ces greffes (Jones JE et Nelson EA. The Cochrane library 2006). De plus, le risque de transmission de maladies virales est maintenu avec l'utilisation de cellules allogéniques.

Il est également possible d'utiliser des kératinocytes autologues en suspension dans une colle biologique, comme le modèle BioSeed®-S, qui est un substitut cutané autologue contenant des kératinocytes autologues adultes mis en suspension dans une colle biologique à base de fibrine (Tissucol^{®}) après 2 à 3 semaines de culture. La fibrine qui stimule la prolifération et la migration des kératinocytes, assure également la fixation des cellules sur la plaie (Clark RAF et al. J Invest Dermatol 1982; Hunyadi J et al. J Dermatol Surg Oncol 1988). Après greffe, les kératinocytes pré-confluents en suspension peuvent former une membrane basale continue avec une jonction dermo-épidermique mieux développée et plus résistante comme cela a été démontré dans un modèle porcin (Chester DL. et al. J Burn Care Rehabil 2004). Cependant, de tels systèmes autologues ne sont disponibles qu'après amplification des cellules du patient et nécessitent donc plusieurs semaines avant de pouvoir être utilisés. Ce délai très long est rédhibitoire dans certaines situations d'urgence (recouvrement cutanés post-chirurgicaux ou brûlures).

Par ailleurs, il n'existe aucune preuve à ce jour qu'un seul des pansements disponibles contenant des cellules de nouveau-nés ou d'adultes (autologues ou non) accélère effectivement la cicatrisation des plaies.

L'utilisation de cellules foetales pour le traitement des déficits cutanés présentent de nombreux avantages par rapport aux cellules adultes. En effet, la réparation cutanée chez le foetus de moins de 24 semaines de gestation est suivie d'une cicatrisation rapide sans cicatrice contrairement à la peau adulte (Bullard KM et al. World J Surg 2003; Hohlfeld J, et al. Lancet 2005). Ce phénomène apparaît être inhérent à la peau foetale de moins de 24 semaines et est indépendant de l'environnement intra-utérin. Ces propriétés, uniques des cellules foetales, sont dues au profil des cytokines sécrétées et à l'expression génique de ces cellules (Bullard KM et al. World J Surg 2003). L'infiltrat inflammatoire est également moins important et la tolérance des cellules foetales meilleure.

Il n'existe actuellement qu'un seul modèle de substitut cutané à base de cellules de peau foetale (Neocutis^{®}). Ce substitut cutané contient une quantité fixe de cellules foetales humaines provenant d'une banque cellulaire unique, constituée d'un mélange de 90 % de fibroblastes et 10 % de kératinocytes. Or les présents inventeurs ont constaté dans un modèle de cicatrisation *in vitro* qu'un ratio entre cellules kératinocytaires et fibroblastiques foetales allant de 0,75 à 2,5, préférentiellement de 1:1 (50 % kératinocytes et 50 % fibroblastes), voire même de 7 :3 (70 % kératinocytes et 30 % fibroblastes), apparaissait optimal pour la cicatrisation.

Dans ce contexte, les présents inventeurs ont recherché un système qui, i) par le contrôle du ratio kératinocytes/fibroblastes foetaux permet d'optimiser la cicatrisation en maîtrisant notamment la quantité et la nature des facteurs de croissance et des cytokines sécrétés ii) par la nature des cellules foetales garantit une bonne immunotolérance et une diminution de l'inflammation locale.

Grâce à deux banques cellulaires distinctes constituées spécifiquement de kératinocytes ou de fibroblastes humains foetaux, les inventeurs ont démontré par un test *in vitro* que l'utilisation de ces cellules dans un ratio d'environ 1:1, ou d'environ 7 :3, permettait d'obtenir une cicatrisation optimale. Ces ratios ont donc été retenus pour le traitement des plaies et des déficits cutanés. L'application des cellules foetales en de telles proportions stimule les cellules kératinocytaires et fibroblastiques du receveur grâce à la sécrétion (par les cellules foetales) de facteurs cytokinines spécifiques. De plus, l'origine foetale des cellules du système, par la production de cytokines spécifiques limite fortement le risque de rejet.

### Figures

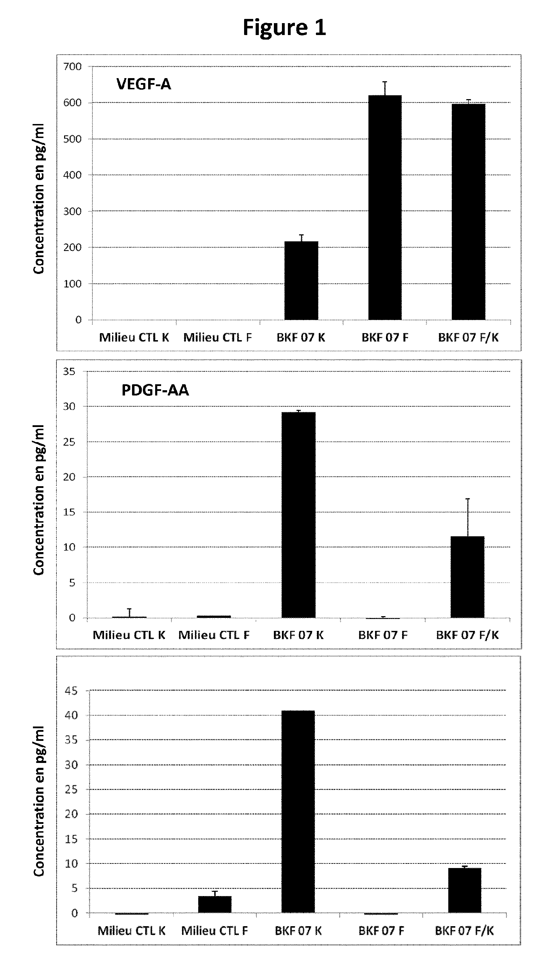

La **figure 1** représente la production des facteurs VEGF A (graphique du haut), PDGF-AA (graphique du milieu) et de l'IL-1ß (graphique du bas) dans le surnageant de cellules kératinocytaires et fibroblastiques foetales obtenues du donneur BKF 07, et dans le surnageant du mélange au ratio 1:1 de cellules fibroblastiques et kératinocytaires du même donneur.

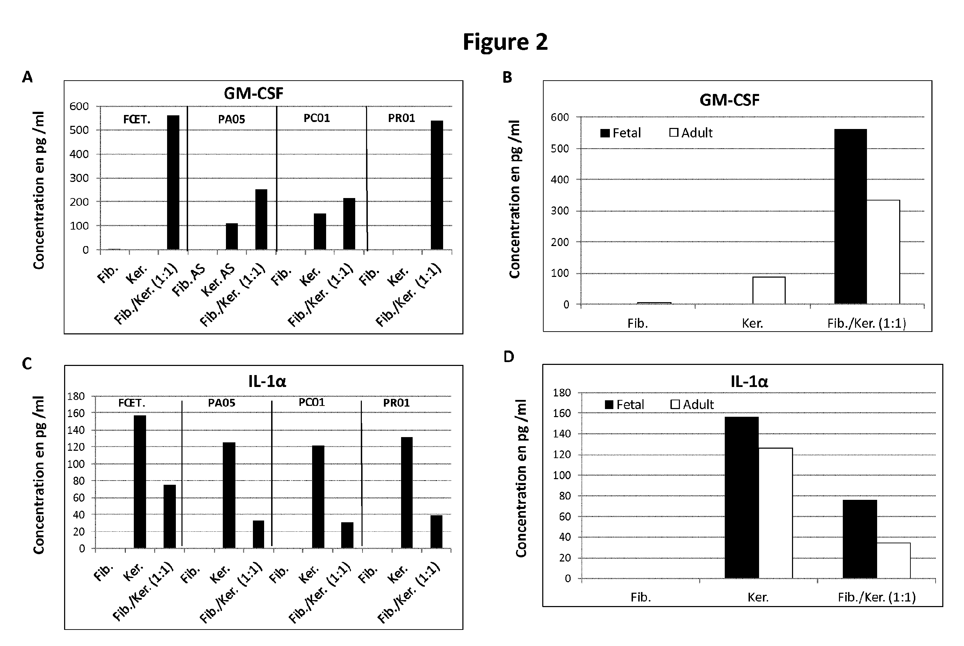

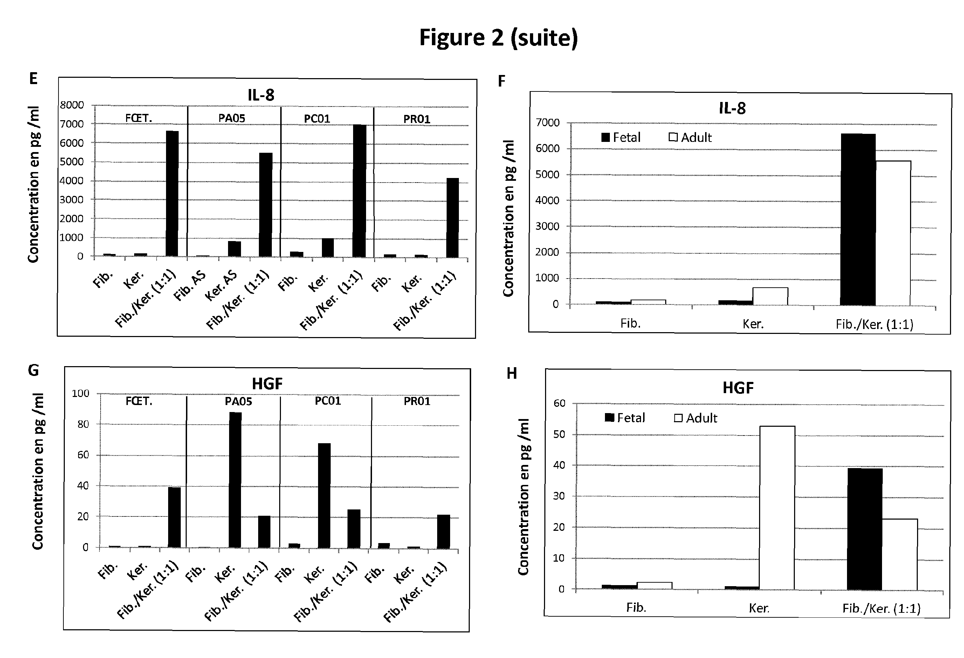

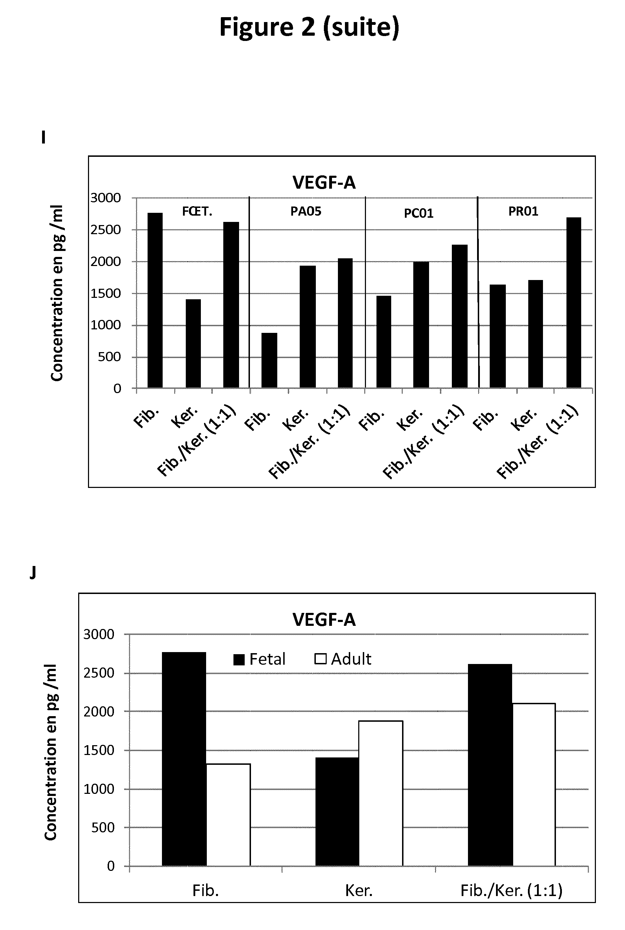

La **figure 2** représente la production des facteurs GM-CSF (2A et 2B), IL-1α (2C et 2D), IL-8 (2E et 2F), HGF (2G et 2H), et VEGF-A (21 et 2J) dans le surnageant de cellules kératinocytaires et fibroblastiques foetales obtenues du donneur BKF 07, seules ou mélangées au ratio 1:1, ainsi que dans le surnageant de cellules kératinocytaires et fibroblastiques adultes, seules ou mélangées au ratio 1 :1 (PA : cellules issues d'une plastie abdominale d'un donneur adulte; PC : cellules issues d'une plastie de la cuisse d'un donneur adulte; PR 01: cellules issues du prépuce d'un donneur adulte). Pour chaque cytokine, deux types de graphiques sont présentés. Les graphiques A, C, E, G et I représentent les résultats des dosages de cytokines pour chaque échantillon de donneur testé, tandis que les graphiques B, D, F, H et J représentent la comparaison de la production des cytokines de la moyenne des échantillons de cellules adultes à celle des cellules d'origine foetale.

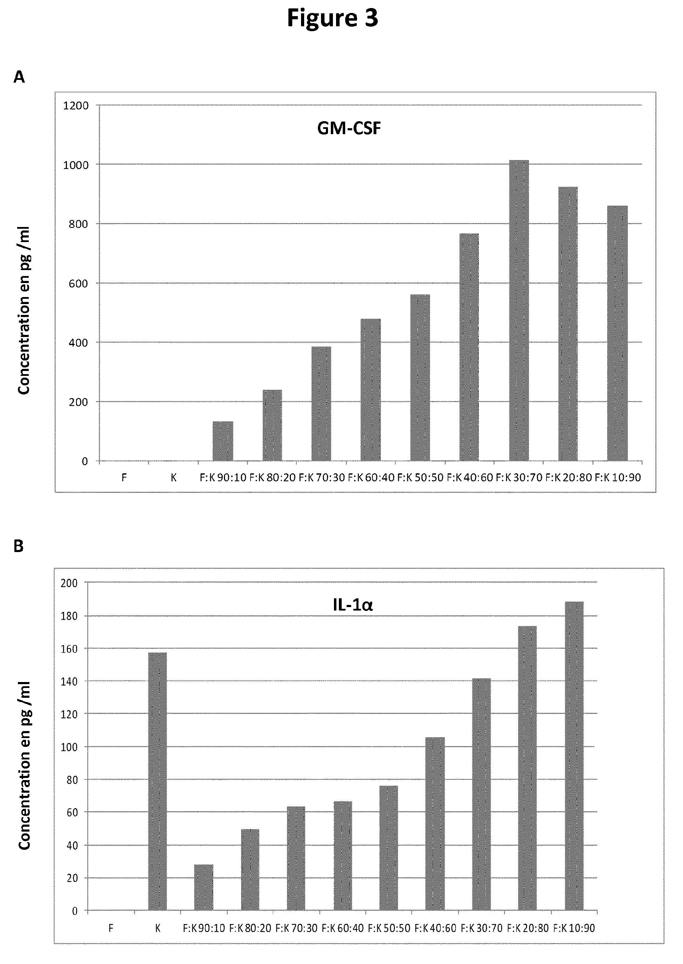

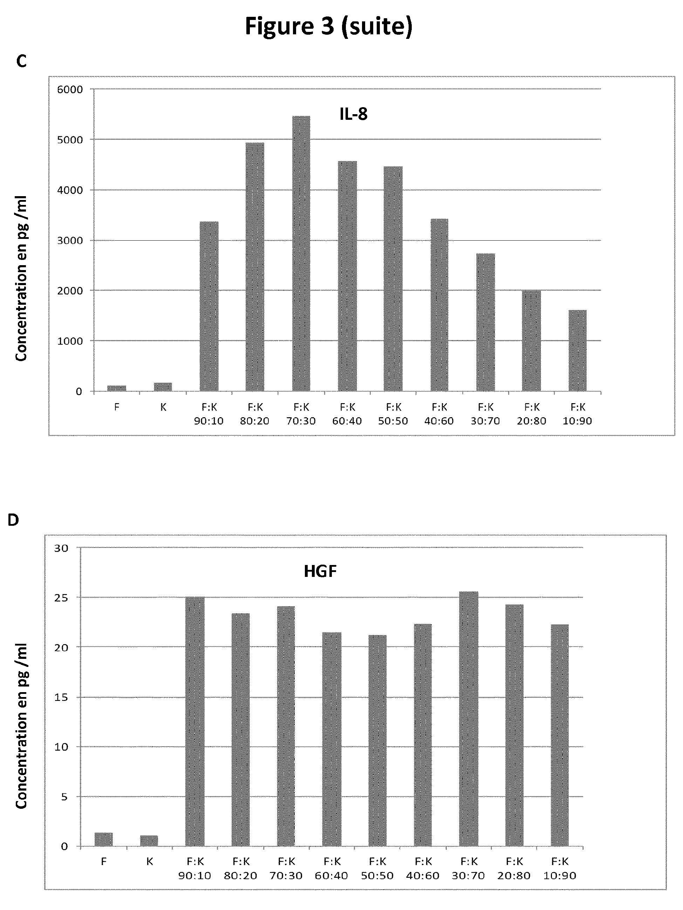

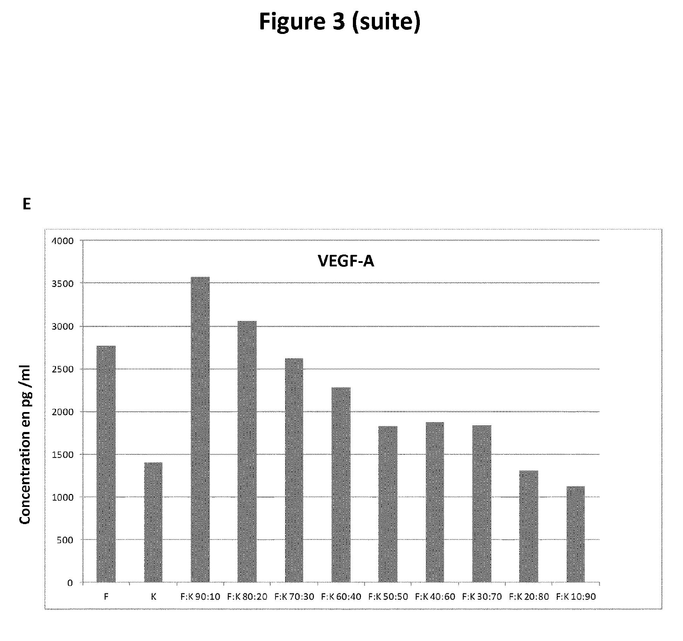

La **figure 3** représente la production de GM-CSF (3A), IL-1α (3B), IL-8 (3C), HGF (3D), et VEGF-A (3E) dans le surnageant de cellules kératinocytaires (K) et fibroblastiques (F) foetales, seules ou mélangées à différents ratios.

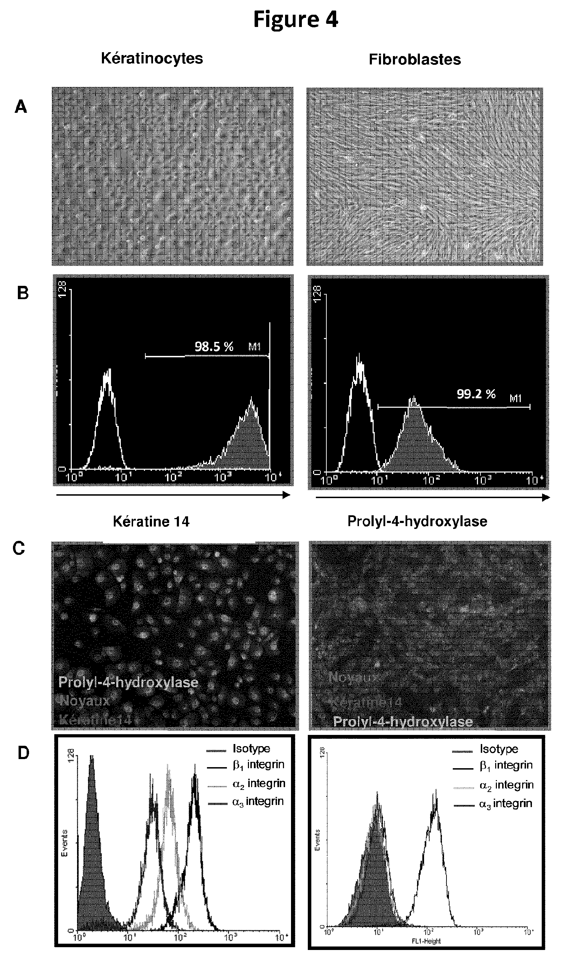

La **figure 4** représente la caractérisation des cellules foetales kératinocytaires et fibroblastiques obtenues à partir du donneur BKF 07. **A**, Photographie de microscopie à contraste de phase des cellules en culture confluente provenant des lots cliniques de kératinocytes (image de gauche) et fibroblastes (image de droite). **B**, Histogramme de cytométrie indiquant le pourcentage de cellules exprimant la kératine 14 au sein du lot clinique de kératinocytes (histogramme de gauche) et le pourcentage de cellules exprimant la prolyl-4-hydroxylase au sein du lot clinique de fibroblastes (image de droite). **C**, Photographie de microscopie à fluorescence indiquant l'expression de la kératine 14 et de la prolyl-4-hydroxylase sur des tapis cellulaires obtenus après décongélation de cellules du lot clinique de kératinocytes (image de gauche) et du lot clinique de fibroblastes (image de droite). **D**, Cytogrammes mettant en évidence l'expression des intégrines ß1, α2 et α3 par les kératinocytes (cytogramme de gauche) et les fibroblastes foetaux (cytogramme de droite). Ils mettent en évidence que les kératinocytes expriment fortement les intégrines ß1, α2 et α3 alors que les fibroblastes n'expriment que l'intégrine ß1.

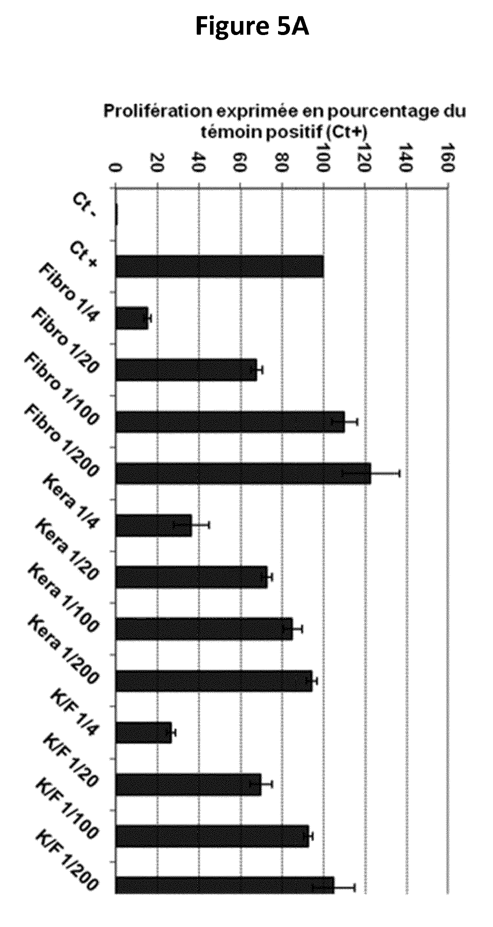

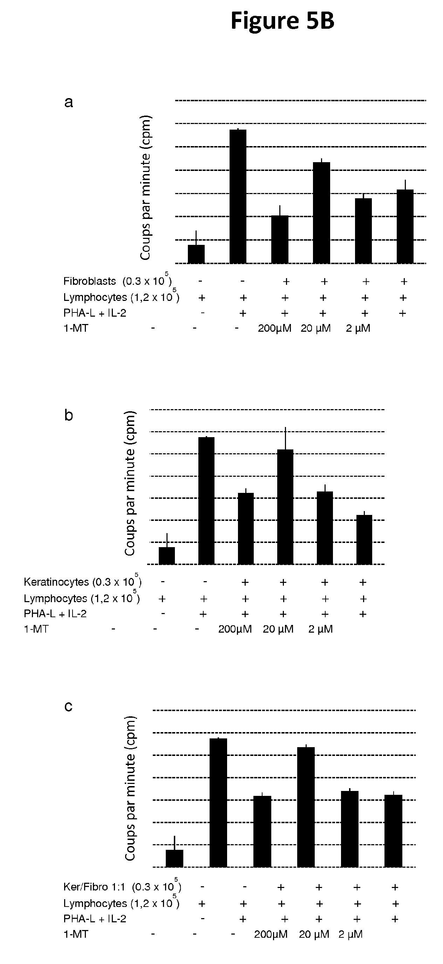

La **figure 5** représente (A) l'inhibition de la prolifération de cellules monocytiques du sang périphérique (PBMC) par les cellules foetales fibroblastiques seules (ratio cellules fibroblastiques / PBMC : 1/4, 1/20, 1/100 et 1/200), par les cellules foetales kératinocytaires seules (ratio cellules kératinocytaires / PBMC : 1/4, 1/20, 1/100 et 1/200), ou par le mélange de cellules kératinocytaires et fibroblastiques 1 :1 (ratio cellules fibroblastiques et kératinocytaires / PBMC :1/4, 1/20, 1/100 et 1/200). Pour les différentes conditions, les résultats sont exprimés en pourcentage de la prolifération par rapport au contrôle positif (PBMC stimulés avec PHA-L et IL-2 sans cellules foetales). (B) représente l'inhibition de la prolifération lymphocytaire par les cellules foetales fibroblastiques seules (ratio cellules fibroblastiques / PBMC : 1/4)(a), par les cellules kératinocytaires seules (ratio cellules kératinocytaires / PBMC : 1/4)(b), ou par le mélange de cellules kératinocytaires et fibroblastiques 1:1 (ratio cellules fibroblastiques et kératinocytaires / PBMC : 1/4)(c) en présence ou non de l'inhibiteur spécifique de IDO (indoléamine 2,3 dioxygénase,) le 1-MT (1-méthyl tryptophane) utilisés aux concentrations 200 µM, 20µM et 2µM.

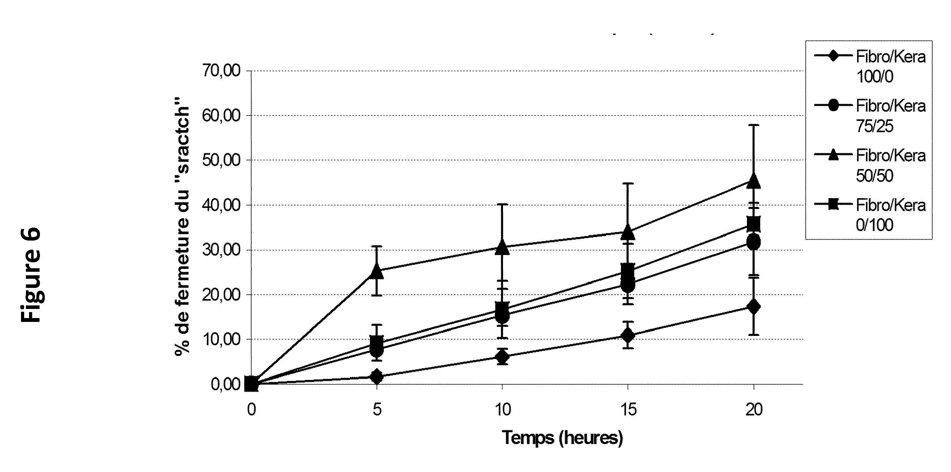

La **figure 6** représente l'évolution de la fermeture d'un scratch cellulaire au cours du temps (entre 0 et 20 heures après le scratch), pour différentes conditions cellulaires (ratio fibroblastes / kératinocytes de 100/0, 75/25, 50/50 ou 0/100).

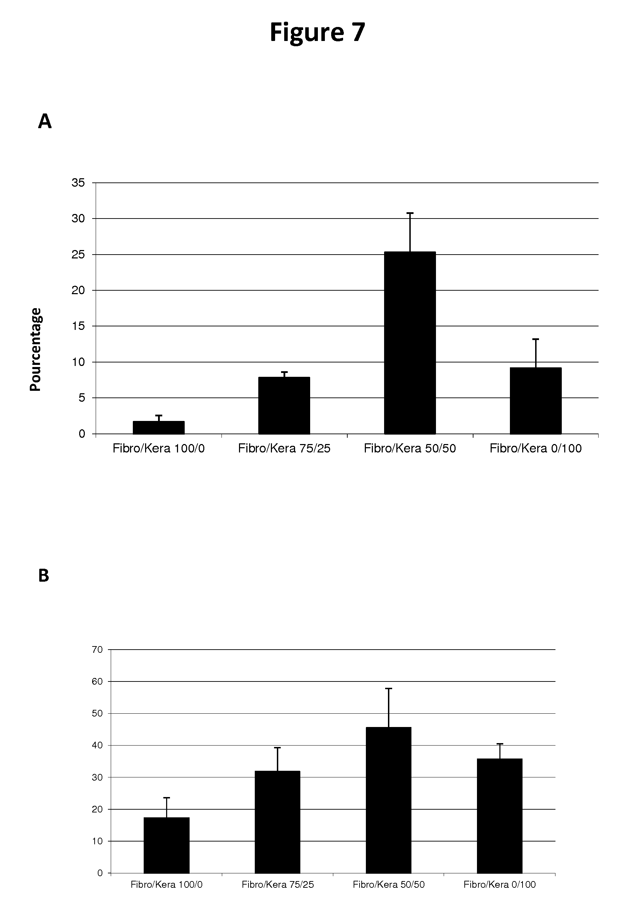

La **figure 7** représente le pourcentage de fermeture du scratch 5 heures (A) ou 20 heures (B) après que celui-ci a été réalisé, pour les différentes conditions cellulaires (ratio fibroblastes / kératinocytes de 100/0, 75/25, 50/50 ou 0/100).

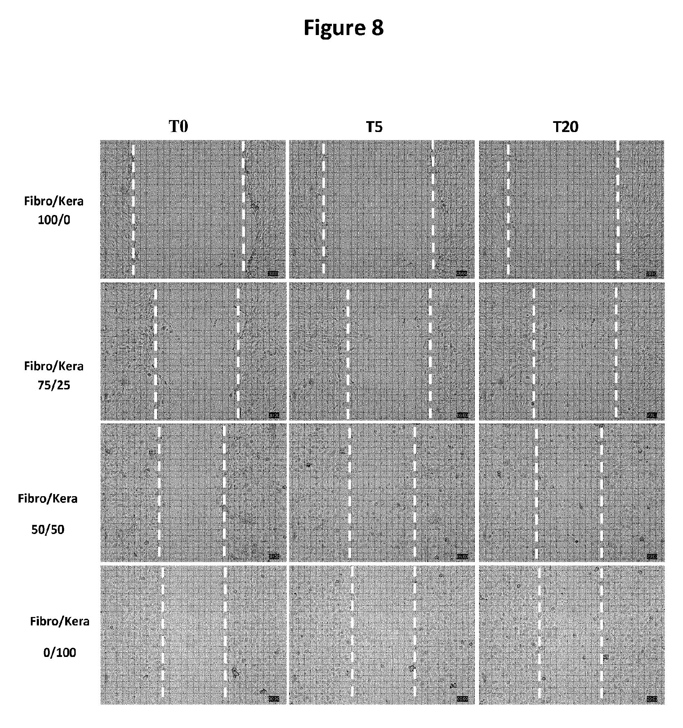

La **figure 8** représente des images du scratch obtenues par microscopie à différents temps (T0, T5 et T20 heures) pour différentes conditions cellulaires (ratio fibroblastes / kératinocytes de 100/0, 75/25, 50/50 ou 0/100).

### Description détaillée de l'invention

Les inventeurs ont, pour la première fois, constitué deux banques de cellules foetales humaines distinctes, l'une contenant des cellules kératinocytaires humaines foetales, l'autre contenant de cellules fibroblastiques humaines foetales. L'élaboration de ces deux banques leur a permis d'étudier l'influence de chacun de ces types cellulaires sur la cicatrisation de blessures cutanées. Elle leur a ainsi permis de mettre au point une composition dans laquelle la proportion de fibroblastes et / ou kératinocytes foetaux humains est précisément adaptée pour stimuler efficacement la cicatrisation cutanée, notamment, via la sécrétion de facteurs de croissance produits par les fibroblastes et les kératinocytes foetaux.

L'originalité de cette composition repose sur la proportion particulière de fibroblastes et / ou kératinocytes foetaux humains identifiée par les inventeurs, qui n'avait jamais été proposée à ce jour. Cette proportion particulière permet d'accélérer le processus de cicatrisation.

Les avantages d'une telle composition sont nombreux:
D'une part, d'un point de vue purement pratique, cette composition ne nécessite pas d'épiderme différencié ou de jonction dermo-épidermique, et elle peut être mise à la disposition d'un patient dans les 48 à 72 heures suivant sa demande. De plus, chaque composante (kératinocytes ou fibroblastes) de la composition est facilement transportable à distance, de préférence en ampoules congelées dans un container en vapeur d'azote.
D'autre part l'origine foetale des cellules qui y sont présentes confère à cette composition une parfaite tolérance et un risque de rejet minime.

Enfin, d'un point de vue thérapeutique, les cellules foetales présentes dans cette composition sécrètent en quantité importante des facteurs cytokiniques (par exemple PDGF-AA, VEGF-A, GM-CSF, HGF) qui ont un rôle essentiel dans la cicatrisation en stimulant efficacement l'angiogenèse, la migration et la prolifération cellulaire, présageant d'une cicatrisation rapide et d'excellente qualité.

La présente invention vise donc, dans un premier aspect, une composition comprenant un mélange de cellules kératinocytaires humaines foetales et de cellules fibroblastiques humaines foetales, le ratio entre lesdites cellules kératinocytaires et fibroblastiques allant de 0,75 à 2,5. Selon un mode de réalisation préféré de l'invention, ledit ratio est compris entre 0,75 et 1,25, et plus préférentiellement est de 1:1. Selon un autre mode de réalisation préféré, ledit ratio va de 1,25 à 2,5, et plus préférentiellement est de 7 :3.

Au sens de la présente invention, le terme « composition » désigne un mélange de cellules kératinocytaires foetales humaines et de cellules fibroblastiques foetales humaines, suspendues ou intégrées dans un milieu non-immunogène adapté à leur survie et à leur migration (par exemple le collagène, la fibrine, le chitosan), ledit milieu favorisant (ou en tous cas n'empêchant pas) la sécrétion des cytokines et facteurs de croissance naturellement sécrété(e)s par lesdites cellules, qui sont notamment le « Vascular Endothelial Growth Factor » (VEGF), le « Platelet-Derived Growth Factor » PDGF-AA, et l'interleukine 1 - bêta (IL1β). Ledit milieu favorise également (ou en tout cas n'empêche pas) le pouvoir immunomodulateur exercé par lesdites cellules. En particulier, cette composition peut être considérée comme un substitut cutané, puisque, lorsqu'elle est appliquée sur une blessure, une plaie, une brûlure, ou un déficit cutané chez un sujet animal ou humain, elle favorise la régénération de la peau endommagée en i) migrant rapidement vers la lésion pour y reconstituer le derme et/ou l'épiderme, ii) influant positivement sur la migration des cellules du sujet via les facteurs de croissance qu'elles sécrètent et iii) limitant l'inflammation locale grâce à ses propriétés immunosuppressives.

Au sens de la présente invention, on entend par « cellules kératinocytaires humaines foetales » des cellules kératinocytaires d'origine foetale, i.e., obtenues à partir de peau foetale humaine, lesdites cellules ayant les caractéristiques spécifiques suivantes : i) une morphologie typique des cellules épithéliales, ii) une propension à sécréter des facteurs de croissance et cytokines essentiels dans le processus de cicatrisation, iii) des propriétés tolérogènes et iv) une forte capacité de migration. Avantageusement, les cellules kératinocytaires humaines foetales utilisées dans la composition de l'invention sécrètent des cytokines et des facteurs de croissance spécifiques des kératinocytes (par exemple le PDFG-AA et l'IL-1β), expriment un ou plusieurs marqueur(s) connu(s) des kératinocytes précurseurs (par exemple la kératine 14, la kératine 5, ou la kératine 15), et présentent des propriétés fonctionnelles migratoires (par exemple expression des intégrines β1, α2 et α3) et d'immunosuppression liées à leur origine foetale et reposant sur l'activité enzymatique Indoléamine-2,3-dioxygénase (IDO) (cf. figure 5B b). Ces caractéristiques spécifiques peuvent être mises en évidence par toute technique classiquement utilisée à cet effet, et notamment par microscopie conventionnelle, dosage Elisa, cytométrie en flux (FACS), immunofluorescence, et test de prolifération lymphocytaire.

Ces cellules kératinocytaires foetales peuvent être obtenues par tout procédé connu de l'homme du métier permettant de conserver les propriétés sus-citées. Notamment, elles peuvent être obtenues en mettant en culture des explants de peau foetale humaine permettant la sortie des kératinocytes des explants puis leur prolifération dans un milieu approprié. De tels milieux sont très largement décrits dans la littérature. Il est possible d'utiliser par exemple le milieu CnT-07 commercialisé par CELLnTEC, le milieu KGM2 commercialisé par Promocell, le milieu KSFM commercialisé par Invitrogen, éventuellement supplémenté en antibiotiques classiques pour éviter la contamination par des bactéries pendant l'étape de culture cellulaire.

Pour générer des cellules kératinocytaires foetales humaines utilisables dans la composition de l'invention, les inventeurs utilisent avantageusement un protocole particulier permettant d'obtenir séparément, à partir du même échantillon de peau foetale humaine, des cellules kératinocytaires et des cellules fibroblastiques humaines foetales (cf. exemple I, section 1.3). Ce protocole est décrit dans l'exemple I+III ci-dessous. Pour résumer brièvement, le prélèvement de peau foetale a été rincé par immersion dans un milieu de culture salé tamponné (par exemple le milieu « phosphate buffered saline » (PBS) ou le milieu « Dulbecco's phosphate buffered saline » (DPBS)), puis découpé en explants de petite taille (environ 1 mm de côté) qui ont été placés dans des plaques de culture, puis ont été recouverts d'un milieu de culture classique (par exemple du MEM ou du DMEM complémenté avec 20% de sérum de veau foetal et 1% de pénicilline-streptomycine) avant d'être incubés à 37°C en atmosphère humide avec 5% de CO₂ pendant en moyenne 10 à 15 jours. Un changement de milieu a été effectué par retrait de la moitié du milieu et rajout de l'équivalent en milieu frais après 5 jours de culture. Selon ce procédé particulier, les fibroblastes sont décollés par action enzymatique (trypsine EDTA) pendant un temps court de 5 à 10 minutes de sorte que seuls les fibroblastes se détachent tandis que les kératinocytes restent adhérents. Les fibroblastes en suspension sont alors transférés dans un tube, centrifugés et remis en culture dans un flacon de culture. Les puits ne contenant plus que les kératinocytes toujours adhérents sont rincés avec un milieu qui inhibe l'action de la trypsine (par exemple DMEM, SVF 10%). Après un rinçage des puits au DPBS, la croissance des kératinocytes est favorisée en remplaçant le milieu de culture initial par un milieu de culture approprié à la prolifération des kératinocytes (par exemple le milieu CnT-07) supplémenté en antibiotiques. Après 5 à 7 jours de culture à 37°C en atmosphère humide avec 5% de CO₂ après l'élimination des fibroblastes, les cellules kératinocytaires sont détachées par action de la trypsine puis ensemencées en flacon de culture. Les cellules sont incubées 5 à 7 jours supplémentaires puis détachées par action de la trypsine et enfin congelées à P1 pour constituer une banque initiale de cellules kératinocytaires. Dans un mode particulier de réalisation de l'invention, un aliquote de cette banque initiale est décongelé, les cellules kératinocytaires sont amplifiées à 37°C en atmosphère humide avec 5% de CO₂ jusqu'au troisième passage, P3 (une vingtaine de jours de culture après décongélation de cellules issues de P1), puis congelées pour constituer un lot clinique (ou « banque de cellules ») de cellules kératinocytaires foetales d'intérêt utilisables dans la composition de l'invention.

Ces cellules kératinocytaires foetales peuvent être congelées par tout procédé connu de l'homme du métier utilisant par exemple une solution de congélation composée d'une solution protéique (par exemple Albumine Humaine 40 mg/mL) associée à un cryoformulateur (par exemple Dyméthyl Sulfoxide) au ratio 9 :1.

Une fois ce lot clinique constitué, les cellules kératinocytaires foetales humaines intégrées dans la composition de l'invention n'ont plus besoin d'être générées à partir de peau foetale extemporanément car elles peuvent être obtenues directement à partir du lot clinique de cellules kératinocytaires foetales humaines, en remettant en culture un échantillon de cellules kératinocytaires foetales humaines dans un milieu approprié (par exemple le milieu CnT-07 sus-mentionné).

Au sens de la présente invention, on entend par « cellules fibroblastiques humaines foetales » des cellules fibroblastiques d'origine foetale, i.e., obtenues à partir de peau foetale humaine, lesdites cellules ayant les caractéristiques spécifiques suivantes : i) une morphologie typique de cellules fibroblastiques, ii) une propension à sécréter des facteurs de croissance et cytokines essentiels dans le processus de cicatrisation, iii) des propriétés tolérogènes et iv) une forte capacité de migration. Avantageusement, les cellules fibroblastiques humaines foetales utilisées dans la composition de l'invention sécrètent fortement des cytokines et des facteurs de croissance tel que le VEGF-A, et expriment un ou plusieurs marqueur(s) connu(s) des fibroblastes comme la prolyl-4-hydroxylase. Ils présentent des propriétés fonctionnelles migratoires (par exemple l'expression de l'intégrine β1), et d'immunosuppression liées à leur origine foetale et reposant sur l'activité enzymatique Indoléamine 2,3 dioxygénase (IDO) (cf. figure 5B a). Ces caractéristiques spécifiques peuvent être misent en évidence par toute technique classiquement utilisée à cet effet, et notamment par microscopie conventionnelle, dosage Elisa, cytométrie en flux (FACS), immunofluorescence, et test de prolifération lymphocytaire.

Ces cellules fibroblastiques foetales peuvent être obtenues par tout procédé connu de l'homme du métier permettant de conserver les propriétés sus-citées. Notamment, elles peuvent être obtenues en mettant en culture des explants de peau foetale humaine permettant la sortie des cellules fibroblastiques des explants puis leur prolifération dans un milieu approprié. De tels milieux sont très largement décrits dans la littérature. Il est possible d'utiliser par exemple, le MEM ou le DMEM supplémenté de 20% de SVF et d'antibiotiques classiques pour éviter la contamination par des bactéries pendant l'étape de culture cellulaire.

Il est également possible d'utiliser le protocole particulier décrit dans l'exemple I (section 1.2) mentionné ci-dessous. Selon ce protocole, qui permet d'obtenir à la fois des fibroblastes et des kératinocytes à partir d'un même échantillon de peau foetale, les cellules fibroblastiques foetales sont obtenues en rinçant ledit prélèvement de peau foetale humaine dans un milieu de culture salé tamponné (par exemple le milieu PBS ou le DPBS), puis en plaçant des explants de peau sur des plaques de culture qui sont ensuite recouvertes d'un milieu de culture classique (par exemple du DMEM complémenté avec 20% de sérum de veau foetal et 1% de pénicilline-streptomycine) avant d'être incubés à 37°C en atmosphère humide avec 5% de CO₂ pendant en moyenne 10 à 15 jours. Un changement de milieu est effectué par retrait de la moitié du milieu et rajout de l'équivalent en milieu frais après 5 jours de culture. Selon ce procédé particulier, les fibroblastes sont décollés par action enzymatique (trypsine EDTA) pendant un temps court, de 5 à 10 minutes, de sorte que seuls les fibroblastes se détachent tandis que les kératinocytes restent adhérents. Les fibroblastes en suspension sont alors transférés dans un tube, centrifugés et remis en culture à 37°C/5% CO₂ en flacon dans un milieu classique, tel que le DMEM supplémenté de 10% de SVF. Un changement de milieu est effectué par retrait de la moitié du milieu et rajout de l'équivalent en milieu frais tous les 3 à 4 jours. Après 10 à 15 jours de culture, les cellules sont détachées par action de trypsine-DETA puis congelées à P1 pour constituer une banque initiale de cellules fibroblastiques. Dans un mode particulier de réalisation de l'invention, un échantillon de cette banque initiale est décongelé, les cellules fibroblastiques sont amplifiées à 37°C en atmosphère humide avec 5% de CO₂ jusqu'au troisième passage (P3, une vingtaine de jours de culture après décongélation de cellules issues de P1), puis congelées pour constituer un lot clinique de cellules fibroblastiques foetales d'intérêt utilisables dans la composition de l'invention.

Ces cellules fibroblastiques foetales peuvent être congelées par tout procédé connu de l'homme du métier utilisant par exemple une solution de congélation composée d'une solution protéique (par exemple Albumine Humaine 40 mg/mL) associée à un cryoformulateur (par exemple Dyméthyl Sulfoxide) au ratio 9 :1.

Une fois le lot clinique constitué, les cellules fibroblastiques foetales humaines intégrées dans la composition de l'invention n'ont plus besoin d'être générées à partir de peau foetale extemporanément car elles peuvent être obtenues directement à partir du lot clinique de cellules fibroblastiques foetales humaines constitué, en remettant en culture un échantillon de cellules fibroblastiques foetales humaines dans un milieu approprié (par exemple le milieu DMEM susmentionné).

De préférence, la peau utilisée pour produire les cellules kératinocytaires et fibroblastiques utilisées dans la composition de l'invention est prélevée sur un foetus humain âgé de 18 à 24 semaines, de manière encore plus préférée sur un foetus humain âgé de 18 à 20 semaines.

Les cellules kératinocytaires et fibroblastiques foetales humaines doivent être présentes dans la composition de l'invention selon des proportions bien précises pour induire une cicatrisation efficace et rapide de la lésion cutanée. Ainsi, le ratio entre le nombre de cellules kératinocytaires et le nombre de cellules fibroblastiques (kératinocytes / fibroblastes) dans la composition de l'invention peut aller de 0,75 à 2,5. Dans un mode de réalisation préféré, le ratio entre le nombre de cellules kératinocytaires et le nombre de cellules fibroblastiques (kératinocytes / fibroblastes) est compris entre 0,75 et 1,25, de préférence entre 0,85 et 1,15, et le plus préférentiellement est de 1 :1 (moyennant l'erreur due à la numération cellulaire). Dans un mode de réalisation préféré alternatif, le ratio entre le nombre de cellules kératinocytaires et le nombre de cellules fibroblastiques (kératinocytes / fibroblastes) va de 1,25 à 2,5, de préférence de 1,5 à 2,5, de 1,75 à 2,5, de 2 à 2,5, de 2,25 à 2,5, et le plus préférentiellement est de 7 :3 (moyennant l'erreur due à la numération cellulaire).

Les techniques de comptage de cellules sont aujourd'hui très bien décrites et sont fiables à quelques pourcents près (typiquement, de 2 à 5%). Il est possible d'utiliser par exemple des compteurs automatiques de cellules (notamment le système « Cellcounter » commercialisé par Millipore, Invitrogen ou Biorad) ou de compter les cellules manuellement, en utilisant des cellules de numération dédiées (cellules de Malassez, de Thoma). Il est important d'utiliser le même système de comptage pour déterminer la quantité de chaque type cellulaire pour que l'erreur due à la technique de numération soit la même.

Dans un mode de réalisation particulier, la composition de l'invention contient, outre des cellules kératinocytaires et fibroblastiques humaines foetales, une matrice extracellulaire naturelle ou synthétique, non-immunogène, de préférence pour l'homme. Plus précisément, les cellules kératinocytaires et fibroblastiques humaines foetales de la composition de l'invention sont intégrées dans une matrice extracellulaire constituée de collagène de type I d'origine bovine. Il est à noter que le collagène bovin a été accepté par les agences réglementaires françaises pour une utilisation clinique chez l'homme. En effet, ce type de matrice xénogénique n'induit pas de réponse immunitaire chez le patient du fait de la faible immunogénicité du collagène.

Le terme « matrice extracellulaire » désigne ici un ensemble de macromolécules extracellulaires naturellement présentes dans le tissu conjonctif et des autres tissus des organismes multicellulaires. Cette matrice est constituée en grande partie de glycoprotéines et de protéines pures, ainsi que de glycosaminoglycanes, qui forme un réseau matriciel. Elle joue un rôle dans le soutien structural, l'adhérence, le mouvement et la régulation des cellules au sein d'un tissu. Les protéines de la matrice extracellulaire naturelle sont plus précisément le collagène, l'élastine, la fibronectine, la laminine, les protéoglycanes, la vitronectine, la thrombospondine, la tenascine (cytoactine), l'entactine (nidogene), l'ostéonectine (SPARC), l'anchorine CII, la chondronectine, l'épinectine, l'hyaluonectine, le composant P amyloide, la fibrilline, la mérosine, la laminine S, l'unduline, l'épilligrine et la kalinine.

Par matrice « non-immunogène », on entend ici que cette matrice est constituée de composés qui n'induisent pas (ou très peu) de réponse immunitaire chez l'individu à qui la composition est destinée. De préférence, ladite matrice est non-immunogène pour l'homme. Une matrice « allogénique» pour l'homme signifie qu'elle est constituée essentiellement de protéines ou de composés d'origine humaine. Une matrice « xénogénique » pour l'homme est quant à elle essentiellement constituée de protéines ou de composés d'origine animale (autres qu'humains). Les deux types de matrice peuvent être utilisés dans la composition de l'invention. Néanmoins, dans le cas où une matrice xénogénique est utilisée, il convient de s'assurer que celle-ci est très faiblement immunogène pour l'homme (comme le collagène bovin par exemple).

Des composés polymériques produits artificiellement (autrement appelés « hydrogels ») peuvent également être utilisés. L'homme du métier est à même d'identifier et de choisir quel composé (artificiel ou naturel) est adapté en fonction de l'application thérapeutique souhaitée.

Dans un mode de réalisation préféré, les cellules kératinocytaires et fibroblastiques humaines foetales de la composition de l'invention sont intégrées dans une matrice extracellulaire contenant au moins un composé naturel choisi parmi : le collagène, la fibrine, la laminine, la fibronectine, l'élastine, le chitosan, l'héparine. Dans un mode de réalisation encore plus préféré, les cellules kératinocytaires et fibroblastiques humaines foetales de la composition de l'invention sont intégrées dans une matrice extracellulaire contenant au moins un composé choisi parmi : le collagène, la fibrine, la fibronectine, et l'élastine. Dans un mode de réalisation préféré entre tous, les cellules kératinocytaires et fibroblastiques humaines foetales de la composition de l'invention sont intégrées dans une matrice extracellulaire contenant au moins un composé choisi parmi : le collagène humain, la fibrine humaine, la fibronectine humaine, et l'élastine humaine. Il est également possible d'intégrer les cellules kératinocytaires et fibroblastiques humaines foetales dans une matrice extracellulaire contenant au moins un composé choisi parmi : le collagène bovin, la fibrine bovine, la fibronectine bovine, et l'élastine bovine.

Avantageusement, la colle intralésionnelle appelée « Tissucol » des laboratoires Baxter peut être utilisée comme matrice extracellulaire dans la composition de l'invention. Cette colle contient du fibrinogène humain, du facteur XIII humain, de la fibronectine humaine, du plasminogène, de l'aprotinine bovine et de la thrombine humaine. Plus précisément, cette colle contient, pour 0,5mL de solution reconstituée, 45 mg de fibrinogène humain, 5 UI de facteur XIII de coagulation humain (une unité de facteur XIII correspondant à l'activité contenue dans 1mL de plasma frais), 2,75mg de fibronectine humaine, 0,04mg de plasminogène humain, 0,83 UPE (Unité pharmacopée européeene) d'aprotinine bovine et 250 UI de thrombine humaine. Cette colle intralésionnelle est dotée de propriétés d'hémostase, de collage, d'adhésion et d'étanchéité des tissus et favorise également la cicatrisation des plaies chirurgicales. Elle peut être utilisée chez des patients sous traitement anti-coagulant ou ayant des troubles de l'hémostase. Le mode d'action de cette colle reproduit la dernière phase de la coagulation : le fibrinogène se transforme en fibrine insoluble sous l'action de la thrombine calcique et du facteur XIII. La fibrine ainsi formée adhère physiquement et chimiquement aux tissus traités et présente un fort degré de réticulation. La thrombine, la fibrine et le facteur XIII ont un effet favorisant la prolifération des fibroblastes. L'étape suivante du processus de cicatrisation de la plaie est constituée par la dégradation protéolytique et phagocytaire du réseau de fibrine. La fibrinolyse dépend également des activateurs du plasminogène tissulaire, dont la concentration peut varier selon les organes et les tissus. L'aprotinine ajoutée à la colle de fibrine inhibe la fibrinolyse induite par les protéases plasmatiques et tissulaires pendant une quinzaine de jours, selon la localisation. La dernière étape est la substitution de la couche de fibrine par un tissu conjonctif cicatriciel. Cette colle peut être reconstituée selon les instructions du fabricant.

Il est finalement possible d'utiliser en tant que matrice extracellulaire dans la composition de l'invention du collagène humain ou bovin qui est le constituant principal de la matrice extracellulaire dans le derme et est donc le support naturel des fibroblastes et des kératinocytes. De préférence on utilisera ici du collagène de type I, qui constitue la trame des tissus conjonctifs, et qui est l'élément matriciel prédominant du le tissu dermique.

On dit que les cellules kératinocytaires et fibroblastiques humaines foetales sont « intégrées » dans la matrice extracellulaire lorsqu'elles sont insérées dans le réseau matriciel créé par l'enchevêtrement des composés de cette matrice. En pratique, on obtient une composition dans laquelle des cellules sont intégrées en mélangeant les cellules avec le (ou les) constituant(s) de la matrice. Le protocole varie en fonction de la matrice utilisée. Par exemple, lorsque la colle « tissucol » est utilisée, les cellules fibroblastiques et kératinocytaires humaines foetales peuvent être directement mélangées avec de la thrombine et le facteur XIII, puis le fibrinogène peut être ajouté, l'ensemble pouvant être directement appliqué sur un déficit cutané tel que plaie, une brûlure ou un ulcère et former un gel solide.

Dans un autre mode de réalisation, la composition de l'invention peut être obtenue en ajoutant le milieu de culture contenant les cellules kératinocytaires et fibroblastiques humaines foetales sur la matrice déjà constituée et en attendant que les cellules y pénètrent par migration. Une fois que les cellules ont commencé à se répartir dans la matrice extracellulaire, le milieu de culture adapté à leur croissance va permettre la colonisation de la totalité de la matrice par les cellules.

Dans un second aspect, l'invention vise donc plus généralement un procédé de fabrication d'une composition, comprenant l'étape de mélanger des cellules kératinocytaires humaines foetales et des cellules fibroblastiques humaines foetales à une matrice extracellulaire non-immunogène, dans un ratio allant de 0,75 à 2,5. Les gammes et valeurs préférées dudit ratio décrites ci-dessus s'appliquent *mutatis mutandis* au présent aspect de l'invention.

Dans un mode de réalisation particulier, ce procédé comprend, au préalable de l'étape de mélange, les deux étapes :
a) obtenir des cellules kératinocytaires humaines foetales, et
b) obtenir des cellules fibroblastiques humaines foetales.

Ces cellules ont été décrites ci-dessus.

De préférence, le procédé de l'invention ne comprend pas d'étape de mise en culture desdites cellules entre l'étape(s) d'obtention de celles-ci et l'étape de mélanger les cellules à la matrice extracellulaire. Ainsi, suite à leur obtention, les cellules kératinocytaires et des fibroblastiques humaines foetales peuvent être directement mélangées à la matrice extracellulaire. Ce mode de préparation particulier permet d'accroître la viabilité des cellules de la composition selon l'invention. Selon ce mode de réalisation particulier, ladite matrice extracellulaire est préférentiellement une matrice de collagène tel que le collagène de type I.

De préférence, ces cellules kératinocytaires et fibroblastiques humaines foetales sont obtenues à partir de deux banques distinctes contenant ces cellules. Dans ce cas, le procédé de fabrication de l'invention comprend les deux étapes :
a) obtenir une banque de cellules kératinocytaires humaines foetales, et
b) obtenir une banque de cellules fibroblastiques humaines foetales.

L'homme du métier sait aisément déterminer, par des manipulations de routine, la concentration et/ou la densité adéquates des cellules kératinocytaires et fibroblastiques humaines foetales dans la matrice extracellulaire en fonction de l'application désirée (en effet, certaines blessures ou brûlures peuvent nécessiter une concentration plus ou moins élevée de cellules en fonction de leur degré).

De même, l'homme du métier sait parfaitement établir, au moyen de simples tests de routine, la quantité et/ou la concentration de chaque constituant de la matrice extracellulaire afin d'obtenir le degré de réticulation souhaité (influant sur l'espacement des fibres dans le réseau matriciel, qui ne doit pas gêner la migration des cellules kératinocytaires et fibroblastiques humaines foetales). Les inventeurs ont identifié que lorsque la composition contient une matrice extracellulaire, il est recommandé d'introduire entre 0.1 et 5.10⁶ cellules par mL/cm³ de matrice.

Pour atteindre la quantité idéale de cellules kératinocytaires et fibroblastiques humaines foetales dans la composition de l'invention, il est possible d'introduire directement la quantité totale de cellules souhaitée, ou encore d'introduire une quantité inférieure puis de mettre en culture la composition dans un milieu adapté (par exemple DMEM complémenté en SVF à 10 %) pendant quelques jours afin de permettre aux cellules kératinocytaires et fibroblastiques humaines foetales de migrer et proliférer dans la matrice afin d'obtenir un pansement optimisé avant son application sur la plaie.

L'application de la composition de l'invention sur la peau endommagée (blessée ou brûlée) peut se faire par n'importe quel moyen connu dans l'art n'affectant pas la viabilité et la mobilité des cellules qu'elle contient. Par exemple, la composition de l'invention peut être appliquée sur la plaie à l'aide d'une seringue ou par le biais d'un pansement qui maintient la composition localisée sur la plaie. En fonction de son mode d'administration, la composition de l'invention peut également contenir un excipient pharmaceutiquement acceptable tel qu'un agent antibactérien, un agent anti-fungi, ou tout solvant classiquement utilisé dans les compositions administrables par voie topique. L'homme du métier saura parfaitement déterminer quels excipients peuvent être utilisés dans la composition de l'invention, dans la mesure où ils n'affectent pas la sécrétion des facteurs de croissance et des cytokines ni la mobilité et la prolifération des cellules présentes dans la composition.

La composition de l'invention est de préférence utilisée sur l'homme. Elle peut néanmoins être utilisée dans des applications vétérinaires, notamment sur des animaux domestiques comme le chien, le chat, le cheval, etc.

Dans un troisième aspect, la présente invention concerne également la composition de l'invention pour son utilisation pour traiter tout déficit cutané, comme par exemple une plaie, une brûlure ou un ulcère et/ou régénérer la peau et/ou accélérer la cicatrisation de tout déficit cutané, par exemple d'une plaie, d'une brûlure ou d'un ulcère. L'invention vise également l'utilisation de la composition de l'invention pour la fabrication d'un pansement ou d'un médicament destiné à régénérer la peau, accélérer la cicatrisation ou soigner tout déficit cutané, par exemple une plaie, une brûlure, un ulcère. L'invention vise enfin une méthode pour régénérer la peau, et/ou soigner et/ou accélérer la cicatrisation de tout déficit cutané, par exemple d'une plaie, d'une brûlure ou d'un ulcère comprenant l'application locale de la composition de l'invention sur ledit déficit cutané.

Dans un quatrième aspect, l'invention concerne enfin un pansement contenant la composition de l'invention telle que définie ci-dessus. Par « pansement » on entend ici un dispositif de protection permettant de recouvrir une partie endommagée de la peau.

Dans un mode de réalisation préféré, le pansement de l'invention est stérile et emballé dans un contenant imperméable aux microorganismes. Dans ce pansement, la composition de l'invention est par exemple recouverte d'un plastifiant, qui peut être choisi dans le groupe constitué par : glycérol, propylène glycol, polyéthylène glycol, polypropylène glycol, sorbitol, autres glycols et éther glycols comme des mono- ou diéthers de polyalkylène glycol, des mono- ou diesters de polyalkylène glycol, des glycolates de polyéthylène glycol, éthylène glycol, diéthylène glycol, triéthylène glycol, dipélargonate de propylène glycol et glycérol de polypropylène glycol, sorbitan esters, esters de l'acide citrique et tartarique, agents de surface amphotériques dérivés d'imidazoline, lactames, amides, polyamides, composés d'ammonium quaternaire, esters comme des phtalates, adipates, stéréates, palmitates, sébacates ou myristates et leurs combinaisons, diisopropyl adipate, phtalates et diéthyl sébacate; des hydrocarbures comme la paraffine liquide; le stéaryl alcool éthoxylé, les esters de glycérol, isopropyl myristate, isotridécyl myristate, éthyl laurate, N-méthylpyrrolidone, éthyl oléate, acide oléique, isopropyl adipate, isopropyl palmitate, octyl palmitate, 1,3-butanediol et leurs mélanges.

Dans un autre aspect, la présente invention concerne le pansement de l'invention pour son utilisation pour traiter et/ou accélérer la cicatrisation de tout déficit cutané, par exemple d'une plaie, d'une brûlure ou d'un ulcère. L'invention vise enfin une méthode pour soigner, et/ou régénérer la peau et/ou accélérer la cicatrisation de tout déficit cutané, par exemple d'une plaie, d'une brûlure ou d'un ulcère comprenant l'application locale du pansement de l'invention sur ledit déficit cutané.

Dans un dernier aspect, l'invention vise par ailleurs l'utilisation cosmétique de la composition de l'invention dans un pansement permettant d'induire une cicatrisation sans cicatrice. Cette utilisation se distingue de l'application thérapeutique mentionnée plus haut en ce qu'elle permet de gommer les traces de la blessure une fois la plaie refermée.

### Exemples

### I. Matériel et méthodes

### 1.1. Préparation de l'échantillon de peau foetale

Le travail de sélection des donneurs est assuré par le service de gynécologie-obstétrique du CHU de Nantes. Il consiste à retenir des foetus d'âge gestationnel compris entre 12 et 22 semaines récupérés lors d'interruptions médicales de grossesse. Ces foetus ne doivent présenter aucune anomalie liée à un virus ni d'anomalie chromosomique. La sérologie de la mère doit être négative pour : hépatite C, AgHBs, Ac HBc, HIV 1 et 2, Ag p24, HTLV 1 et 2, CMV.

Les foetus sont récupérés au cours d'interruptions médicales de grossesse sous contrôle échographique, en conditions stériles. L'obstétricien examine le foetus à la recherche de malformations qui conduiraient à son exclusion.

Le prélèvement de peau est effectué en salle d'accouchement par l'obstétricien senior qui place le prélèvement dans un tube stérile vide. Le prélèvement est alors recouvert d'un milieu de culture (DMEM/SVF) contenant des antibiotiques (Pénicilline-Streptomycine).

Le traitement du prélèvement de peau foetale est réalisé sous hotte à flux laminaire en zone à atmosphère contrôlée de l'UTCG. Le prélèvement de peau est rincé par immersion pendant 1 minute dans 2 tubes 50ml successifs contenant 20ml de DPBS / P-S (2%) et agitation. Il est ensuite transféré dans un plateau stérile et étalé derme au-dessus.

La peau, maintenue à l'aide d'une pince, est raclée en surface par une lame de scalpel afin d'en éliminer l'hypoderme. La lame du scalpel est inclinée avec un angle de 45° par rapport à la surface de la peau. La peau est à nouveau rincée par immersion et agitation dans 2 tubes 50ml successifs contenant 20ml de DPBS / P-S (2%).

Le prélèvement de peau foetale est placé dans une boite de Pétri, épiderme vers le haut. 2 fragments de 2 à 3 mm de côté sont découpés dans la peau. Ils sont placés dans un cryotube puis congelés à sec à -80°C. Ces fragments sont destinés à des recherches complémentaires à des fins thérapeutiques.

Le reste de la peau est découpé en explants les plus petits possibles (environ 1mm de coté).

Les explants sont ensuite placés dans des plaques 6 puits, à raison de 10 explants par puits, espacés de façon régulière. Après 10 minutes d'attente, les explants sont recouverts de 750µl de milieu de culture (DMEM complémenté avec 20% de SVF, 1% de P-S). Les plaques 6 puits sont ensuite placées à l'incubateur pour 6h ou une nuit. Après ce délai, 2ml de milieu de culture sont ajoutés dans chaque puits. Les plaques 6 puits sont transférées à l'incubateur à 37°C en atmosphère humide avec 5% de CO₂ pendant 10 à 15 jours en moyenne.

Tous les 3 à 4 jours, une observation microscopique de la culture afin d'évaluer l'émergence des cellules est réalisée. Un changement de milieu est effectué par retrait de la moitié du milieu et rajout de l'équivalent en milieu frais DMEM/ SVF (10%)/ PS (1%).

Après 10 à 15 jours en moyenne, les explants sont entourés d'une couronne de kératinocytes au-delà duquel on retrouve le tapis de fibroblastes. Les fibroblastes et kératinocytes sont alors récupérés séparément, en 2 temps.

### I.2. Production de fibroblastes foetaux

Une première trypsinisation permet l'obtention des fibroblastes. Pour cela, le milieu de culture est retiré des plaques 6 puits à l'aide d'une pipette. Les puits sont ensuite rincés avec 2ml de DPBS.

Le DPBS est retiré de chaque puits et remplacé par 0,5ml de trypsine-EDTA 1X. Les plaques sont incubées à 37°C pendant 5 à 10 min. La trypsine est utilisée à température ambiante afin de permettre aux kératinocytes de rester adhérents alors que les fibroblastes se détachent. Le décollement est vérifié par observation des plaques au microscope.

Les fibroblastes sont alors repris avec 1ml de DMEM/ SVF (10%)/ PS (1%) par puits afin d'inhiber l'action de la trypsine. Les fibroblastes en suspension sont « poolés » dans un tube de 15 ou 50ml qui sera centrifugé. Le surnageant est éliminé et le culot cellulaire est repris dans 1 à 5 ml de milieu DMEM/ SVF (10%)/ PS (1%). Les fibroblastes sont alors ensemencés à raison de 8000 à 12000 cellules par cm² en flacons de culture. Les flacons de culture sont transférés à l'incubateur à 37°C/5% CO₂ pendant 7 à 10 jours.

Tous les 3 à 4 jours, une observation microscopique de la culture afin d'évaluer l'émergence des cellules est réalisée. Un changement de milieu est effectué par retrait de la moitié du milieu et rajout de l'équivalent en milieu frais DMEM/ SVF (10%)/ PS (1%).

Des échantillons sont congelés à ce stade pour former une banque initiale de cellules fibroblastiques foetales en fin de premier passage (P1). Pour la congélation, les cellules sont ajustées à 1 million de cellules par ml dans une solution de congélation constituée d'albumine humaine à 4 % et de dymethyl sulfoxide (DMSO) au ratio 9 :1. Les cellules sont aliquotées dans des cryotubes 2 ml à raison de 1 ml par tube. La congélation de ces cryotubes est réalisée en diminuant la température de 1°C/minutes jusqu'à -80°C puis après 24 à 72 heures, les cryotubes sont transférés en vapeur d'azote entre -130 et -196°C. Un aliquot de la banque initiale est ensuite décongelé, puis les cellules fibroblastiques foetales sont amplifiées 2 passages supplémentaires en milieu DMEM/SVF (10%) et congelées suivant la procédure décrite ci-dessus pour constituer un lot clinique (banque de travail) de cellules fibroblastiques foetales à P3. L'aspect morphologique ainsi que l'expression de la prolyl-4-hydroxylase et des intégrines ß1, α2 et α3 sont contrôlés par microscopie conventionnelle, FACS et immunofluorescence (Figure 4).

### I.3. Production de kératinocytes foetaux

Lorsque les fibroblastes sont récupérés après trypsinisation, les plaques 6 puits sont rincées avec 2mL de DPBS par puits et le milieu de culture initial est remplacé par du milieu CnT-07/ PS (1%) afin de favoriser la prolifération des kératinocytes. Les plaques sont incubées pendant 3 à 7 jours à 37°C /5% CO2.

Tous les 3 à 4 jours, la culture est observée au microscope afin d'évaluer l'émergence des cellules. Un changement de milieu est réalisé par retrait de la moitié du milieu et rajout de l'équivalent en milieu frais CnT-07 / PS (1%).

Dès que la quantité de cellules le permet (confluence 70-80%), les kératinocytes sont décollés et mis en culture. Le milieu de culture est retiré des plaques 6 puits à l'aide d'une pipette. Les puits sont ensuite rincés avec 2 mL de DPBS. Le DPBS est retiré de chaque puits et remplacé par 0,5mL de trypsine-EDTA 1X. Les plaques 6 puits sont incubées à 37°C pendant 10 à 15 minutes. Le décollement est vérifié par observation des plaques au microscope.

Les kératinocytes sont alors repris avec 1ml de DMEM/ SVF (10%)/ PS (1%) par puits afin d'inhiber l'action de la trypsine. Les kératinocytes en suspension sont « poolés » dans un tube de 15 ou 50mL qui sera centrifugé. Le surnageant est éliminé et le culot est repris dans 1 à 5 ml de milieu spécifique des kératinocytes, le CnT-07.

Un prélèvement pour numération/viabilité est effectué.

Les kératinocytes sont ensemencés à raison de 4000 à 6000 cellules par cm² en flacons de culture.

Les flacons de culture sont transférés à l'incubateur à 37°C/5% CO₂ pendant 7 à 10 jours.

Des échantillons sont congelés à ce stade pour former une banque initiale de cellules kératinocytaires foetales en fin de premier passage (P1). Pour la congélation, les cellules sont ajustées à 1 million de cellules par ml dans la solution de congélation constituée d'albumine humaine à 4 % et de dymethyl sulfoxide (DMSO) au ration 9 :1. Les cellules sont aliquotées dans des cryotubes 2 ml à raison de 1 ml par tube. La congélation de ces cryotubes est réalisée en diminuant la température de 1°C/minutes jusqu'à -80°C puis après 24 à 72 heures, les cryotubes sont transférés en vapeur d'azote entre -130 et -196°C. Un aliquot de la banque initiale est ensuite décongelé, puis les cellules kératinocytaires foetales sont amplifiées deux passages supplémentaires en milieu CnT-07 et congelées suivant la procédure décrite ci-dessus pour constituer un lot clinique (banque de travail) de cellules kératinocytaires foetales à P3. L'aspect morphologique ainsi que l'expression de la kératine 14 et des intégrines ß1, α2 et α3 sont contrôlés par microscopie conventionnelle, FACS et immunofluorescence (Figure 4).

### I.4. Profil de sécrétion cytokinique des deux types cellulaires obtenus

Les surnageants de culture de kératinocytes, fibroblastes ou mélange des deux types cellulaires à différents ratios provenant des cultures de type foetal obtenues ci-dessus, ou de cellules adultes, ont été congelés après 48 heures de culture et leur contenu en VEGF-A, PDGF-AA, IL1β, GM-CSF, IL-1α, IL-8 et HGF a été analysé par dosage ELISA.

### I.5. Pouvoir immunomodulateur des fibroblastes et kératinocytes foetaux

Des cellules fibroblastiques et kératinocytaires foetales obtenues comme décrit ci-dessus ont été irradiées puis mises en présence de PBMC stimulés par la phytohématogglutinine-L (PHA-L) et l'Interleukine 2 (IL-2). Différents ratios de cellules foetales / PBMC ont été testés (1/4; 1/20; 1/100; 1/200) de façon à observer un éventuel effet de dose des cellules foetales sur la prolifération lymphocytaire. La prolifération a été évaluée par incorporation de thymidine tritiée après 5 jours de culture.

### I.6. Role de l'IDO (indoléamine,2-3, dioxygénase) sur le pouvoir immunomodulateur des fibroblastes et kératinocytes foetaux.

Le même type d'expérience que celle décrite dans le paragraphe 1.5 a été réalisée à la différence que pour évaluer le rôle de l'IDO sur le pouvoir immunomodulateur des fibroblastes et kératinocytes foetaux, l'inhibiteur spécifique de l'IDO, le 1-MT (1-méthyl tryptophane) a été ajouté aux concentrations 200 µM, 20µM et 2µM durant la phase de prolifération lymphocytaire, de façon à observer une éventuelle réversion de l'effet inhibiteur des cellules foetales sur la prolifération lymphocytaire.

### I.7. Test de cicatrisation in vitro des cellules foetales

Ce test a été réalisé à partir de fibroblastes et de kératinocytes obtenus selon les protocoles mentionnés ci-dessus. Le but de ce test était de déterminer le ratio de chaque type cellulaire permettant d'avoir une cicatrisation optimale. Les cellules ont été ensemencées dans le milieu CnT-07 à une densité proche de la confluence (100 000 cellules par puits d'une plaque 12 puits). Après 24h de culture supplémentaire, le tapis cellulaire a été « scratché » à l'aide d'une pointe (la largeur mesurée du « scratch » est de l'ordre de 300 à 500 µm, voir figure 8). Les cellules ont été lavées au PBS puis du milieu frais non complémenté a été ajouté dans chaque puits. La plaque a été immédiatement analysée en time-lapse (analyse en temps réel de cellules) pendant 23 heures.

Pour l'analyse en time-lapse 4 points de chaque « scratch » ont été étudiés. Pour chacun des points une photo a été réalisée toutes les 10 minutes pendant les 23 heures de l'analyse.

Différentes conditions ont été étudiées, correspondant à 4 tapis cellulaires différents :
1- Fibroblastes seuls (ratio fibro/kera 100/0)
2- 75 % Fibroblastes + 25% kératinocytes (ratio fibro/kera 75/25)
3- 50 % Fibroblastes + 50 % kératinocytes (ratio fibro/kera 50/50)
4- kératinocytes seuls (ratio fibro/kera 0/100).

L'analyse des résultats a été faite à l'aide du logiciel d'image image J. La mesure de la fermeture du scratch a été évaluée par le pourcentage de recouvrement cellulaire de la zone scratchée au temps T0, 5h, 10h, 15h et 20h. Ce pourcentage de recouvrement a été comparé à la moyenne de recouvrement cellulaire des zones à droite et à gauche du scratch (zones de tapis cellulaire) considérée comme le 100% de recouvrement cellulaire.

### II- Résultats

### II.1. Sécrétion de cytokines par les cellules fibroblastiques et kératinocytaires foetales seules ou en mélange (ratio 1 :1)

Les résultats sont présentés sur **la** **figure 1**.

Les cellules foetales, et plus particulièrement les fibroblastes foetaux, sécrètent fortement le VEGF-A. Lors de la co-culture des fibroblastes et des kératinocytes foetaux, le fait d'obtenir une concentration en VEGF-A de même valeur que celle obtenue dans le cas de culture de fibroblastes seuls indique qu'il y a une potentialisation de la sécrétion de VEGF-A entre les deux types cellulaires. En effet, si ce n'était pas le cas, la concentration en VEGF serait plus faible dans le surnageant de la coculture par rapport à celle des fibroblastes seuls, le niveau de sécrétion des kératinocytes étant plus faible.

Les facteurs PDGF-AA et IL-1ß sont spécifiquement sécrétés par les kératinocytes foetaux. Lorsque les fibroblastes et les kératinocytes foetaux sont mélangés au ratio 1 :1, on observe toujours une sécrétion de ces deux facteurs bien que celle-ci soit plus faible que pour les kératinocytes seuls.

Ces dosages montrent bien que chaque type cellulaire sécrète des facteurs de croissance de façon très différente qualitativement et quantitativement. Par ailleurs, ces résultats montrent que l'association de kératinocytes et de fibroblastes (au moins au ratio 1:1) n'empêchent pas la libération des facteurs spécifiques à chacun des deux types cellulaires mais au contraire, pour certains facteurs, cette libération peut-être potentialisée (pour le VEGF-A notamment).

### II.2. Comparaison du profil cytokinique des cellules fibroblastiques et kératinocytaires foetales et adultes, seules ou en mélange (ratio 1 :1)

Les résultats sont présentés sur la **figure 2**.

### • GM-CSF (figures 2A et 2B)

Les Inventeurs ont observé que les fibroblastes et kératinocytes foetaux seuls ne sécrètent pas de GM-CSF. En revanche, lorsque ces cellules sont co-cultivées à un ratio 1 :1, le GM-CSF est fortement surexprimé dans le surnageant de culture (562 pg/ml).

Par ailleurs, quel que soit la source des échantillons de fibroblastes adultes utilisés, ceux-ci ne sécrètent pas de GM-CSF. Les kératinocytes adultes des échantillons PA01 (plastie abodminale) et PC01 (plastie cuisse) sécrètent quant à eux une faible quantité de GM-CSF (110 et 150 pg/ml).

L'échantillon PR01 (prépuce) présente un profil de sécrétion du GM-CSF proche de celui des cellules foetales puisque chaque type cellulaire seul ne sécrète pas de GM-CSF, mais une potentialisation de la sécrétion de cette cytokine est observée en co-culture (538 pg/ml).

Ces résultats démontrent clairement que l'association des fibroblastes et de kératinocytes foetaux permet de potentialiser la sécrétion de GM-CSF, de manière plus importante qu'avec une co-culture de cellules adultes (562 vs 335 pg/ml).

### • IL-1α (figures 2C et 2D)

Les Inventeurs ont observé des profils de sécrétion de l'IL-1α très similaires, ceci quel que soit les échantillons cellulaires testés. Ainsi, il apparaît que les fibroblastes ne sécrètent pas d'IL-1α en monoculture, tandis que les kératinocytes en sécrètent (jusqu'à 157 pg/ml concernant les cellules de type foetal).

Lorsque les fibroblastes et kératinocytes sont en co-culture à un ratio 1 :1, une diminution de la sécrétion de l'IL-1α est observée. Cette diminution est néanmoins beaucoup plus importante chez les cellules adultes que chez les cellules de type foetal.

### • IL-8 (figures 2E et 2F)

Les profils de sécrétion de l'IL-8 sont également très similaires. Ainsi, il apparaît que les fibroblastes et les kératinocytes foetaux n'en sécrètent pas en monoculture. Seuls les kératinocytes adultes issus des prélèvements PA05 (plastie abdominale) et PC01 (plastie cuisse) en sécrètent à des niveaux très faibles (respectivement 870 et 1000 pg/ml).

En revanche, la co-culture des kératinocytes et fibroblastes augmente considérablement la sécrétion d'IL-8, aussi bien pour les cellules foetales (6632 pg/ml) que pour les cellules adultes (5590 pg/ml).

### • HGF (figures 2G et 2H)

En monoculture, ni les fibroblastes ni les kératinocytes foetaux ne sécrètent d'HGF. En revanche, une augmentation importante de la concentration en HGF est observée en coculture (39,5 pg/ml). La comparaison des cellules foetales et adultes en co-culture montre les cellules foetales sécrètent plus fortement le facteur HGF que les cellules adultes (39,5 vs 23 pg/ml). En outre, il apparaît que l'association de fibroblastes et kératinocytes foetaux potentialise fortement la sécrétion d'HGF, alors que l'effet inverse est observé avec les cellules adultes.

### • VEGF-A (figures 2I et 2J)

Les Inventeurs ont observé que les fibroblastes foetaux sécrètent deux fois plus de VEGF-A que les fibroblastes de donneurs adultes (2770 vs 1323 pg/ml). En revanche, la sécrétion de VEGF-A par les kératinocytes adultes est plus importante qu'avec les kératinocytes foetaux (1875 vs 1404 pg/ml).

Par ailleurs, les fibroblastes foetaux sécrètent plus de VEGF-A que les kératinocytes foetaux (2770 vs 1404 pg/ml), à l'inverse des cellules adultes (1875 pg/ml chez les kératinocytes vs 1323 pg/ml chez les fibroblastes).

Enfin, en co-culture, les cellules foetales sécrètent légèrement plus de VEGF-A que les cellules adultes (2617 vs 2100 pg/ml).

### • Conclusion

Ainsi, les Inventeurs ont démontré que la co-culture de fibroblastes et de kératinocytes foetaux est essentielle pour la sécrétion des facteurs GM-CSF, IL-8 et HGF, qui ne sont pas naturellement sécrétés par ces cellules en monoculture. Cette co-culture influence par ailleurs positivement la sécrétion de VEGF-A, ceci de façon modérée.

Les présents résultats démontrent également que, dans des conditions de co-culture (ratio 1 :1), la sécrétion des facteurs GM-CSF, HGF, II-8, IL-1α et VEGF-A est plus importante avec des fibroblastes et kératinocytes d'origine foetale qu'avec des cellules adultes.

### II.3. Profil cytokinique des cellules fibroblastiques et kératinocytaires foetales à différents

### ratios

Afin d'étudier l'influence du ratio de cellules fibroblastiques et kératinocytaires foetales sur la production cytokinique, les Inventeurs ont étudié la concentration de différentes cytokines en fonction de différents ratios de ces cellules.

Les résultats de cette étude sont présentés sur la **figure 3**.

### • GM-CSF (figure 3A)

Les Inventeurs ont observé que c'est l'association de fibroblastes et de kératinocytes foetaux qui permet auxdites cellules d'exprimer le GM-CSF. Ainsi, ces résultats confirment que les interactions entre ces deux types cellulaires sont nécessaires pour la sécrétion de GM-CSF.

En outre, plus la proportion de kératinocytes augmente, plus la sécrétion de GM-CSF s'accroit, jusqu'à atteindre un plateau au ratio 30 % de fibroblastes / 70 % de kératinocytes (1014 pg/ml). La sécrétion de GM-CSF commence ensuite à diminuer lorsque la proportion en kératinocytes dépasse 70%.

### • IL-1α (figure 3B)

Conformément aux résultats du point II.2, on peut noter que seuls les kératinocytes foetaux sécrètent l'IL-1α. Cependant, contrairement au GM-CSF, la co-culture de fibroblastes et kératinocytes foetaux ne semble pas influencer la sécrétion l'IL-1α. Ainsi, au ratio 90 % de fibroblastes / 10 % de kératinocytes, la sécrétion d'IL-1α est très faible et augmente progressivement jusqu'au ratio inverse (10 % de fibroblastes / 90 % de kératinocytes) atteignant alors un niveau légèrement supérieur à celui obtenu pour une monoculture de kératinocytes (188 pg/ml vs. 157 pg/ml). Un ratio de 50% de chaque type cellulaire permet d'atteindre la moitié du niveau obtenu pour une monoculture de kératinocytes.

### • IL-8 (figure 3C)

Comme pour le GM-CSF, les Inventeurs ont observé que c'est l'association de fibroblastes et de kératinocytes foetaux qui induit l'expression d'IL-8. Ainsi, ces résultats confirment que les interactions entre ces deux types cellulaires sont nécessaires pour la sécrétion de cette cytokine.

Le niveau maximal de sécrétion en IL-8 est atteint pour le ratio 70 % fibroblastes / 30 % kératinocytes (5470 pg/ml) mais celui-ci reste cependant relativement stable jusqu'au ratio 50 % / 50 % (4464 pg/ml). Ensuite, plus la proportion en kératinocytes augmente (au-delà de 50%), plus la sécrétion d'IL-8 décroît.

### • HGF (figure 3D)

Comme pour le GM-CSF et l'IL-8, l'association de fibroblastes et de kératinocytes foetaux permet auxdites cellules de sécréter l'HGF. Ainsi, ces résultats confirment que les interactions entre ces deux types cellulaires sont nécessaires pour l'expression de HGF.

En revanche, le ratio entre fibroblastes et kératinocytes foetaux ne semble pas influencer le niveau de sécrétion de HGF (qui varie entre 21 et 25 pg/ml).

### • VEGF-A (figure 3D)

Comme observé au point 11.2, le VEGF-A est la seule cytokine exprimée par les fibroblastes et kératinocytes foetaux en monoculture.

L'association de ces deux types cellulaires influence positivement l'expression de VEGF-A. Ainsi, une concentration maximale en VEGF-A est observée pour le ratio 90% fibroblastes /10 % kératinocytes (3570 pg/ml). Ensuite, plus la proportion en kératinocytes augmente, plus la concentration en VEGF-A diminue.

### • Conclusion

Ces résultats confirment que l'association de fibroblastes et kératinocytes foetaux permet bien la surexpression de plusieurs facteurs de croissance et cytokines, et en particulier du GM-CSF, du HGF et de l'IL8 qui ne sont pas naturellement exprimés dans ces cellules en monoculture et qui sont connus pour leur implication dans le processus de cicatrisation.

Le ratio entre ces deux types cellulaires permet en outre de moduler la sécrétion de ces facteurs et cytokines. Les résultats démontrent notamment que la libération de ces facteurs est de manière générale inférieure avec un ratio fibroblastes/kératinocytes de 9 :1 (WO 03/068287, Néocutis SA), par rapport à un ratio de 1 :1. La concentration en IL-1α, qui est connue pour son rôle dans la cicatrisation, est notamment plus élevée lorsque la proportion en kératinocytes augmente.

Il apparaît en outre qu'un ratio de fibroblastes/kératinocytes de 3 :7 semble être optimal puisqu'il permet d'obtenir une expression maximale de HGF et GM-CSF tout en maintenant une expression optimale d'IL-1α, ce qui est essentiel pour la cicatrisation. Bien que les niveaux d'expression de VEGF-A et d'IL-8 ne soient pas optimaux à ce ratio, ils restent cependant relativement importants. A cet égard, il convient de noter qu'outre une fonction pro-cicatrisante, la cytokine IL-8 présente des propriétés pro-inflammatoires. Ainsi, une trop forte concentration en IL-8 n'est pas souhaitable car celle-ci pourrait conduire à une augmentation de la réponse inflammatoire qui serait néfaste pour la qualité de cicatrisation.

### II.2. Caractérisation des cellules fibroblastiques et kératinocytaires foetales

Les résultats sont présentés dans la **figure 4**.

Ils mettent en évidence que les cellules issues des lots cliniques de kératinocytes et de fibroblastes ont bien une morphologie caractéristique de kératinocytes et de fibroblastes respectivement. La pureté de ces lots cliniques a été validée par cytométrie. Ainsi, 98,5 % des cellules du lot clinique de kératinocytes expriment la kératine 14 (marqueur spécifique des kératinocytes) et 99.2 % des cellules issues du lot clinique de fibroblastes expriment la prolyl-4-hydroxilase (marqueur de fibroblastes). Ces résultats valident le procédé de production retenu. Celui-ci permet bien d'obtenir, deux banques très pures en kératinocytes et fibroblastes à partir d'un prélèvement unique de peau foetale.

### II.3. Inhibition de la lymphoprolifération par les cellules fibroblastiques et kératinocytaires foetales en mélange

Les résultats sont présentés sur la **figure 5**.

Ces résultats mettent en évidence qu'il y a une inhibition de la prolifération lymphocytaire induite par les fibroblastes et les kératinocytes foetaux. Cette inhibition apparaît être dose dépendante et surtout elle est toujours effective lorsque l'on mélange fibroblastes et kératinocytes au même ratio.

Ces résultats démontrent qu'il est avantageux d'utiliser des cellules foetales dans un système allogénique puisqu'elles sont capables d'inhiber l'activation lymphocytaire. Ainsi, en bloquant l'activation lymphocytaire de l'hôte, les cellules foetales non seulement seront bien tolérées et pourront donc jouer leur rôle accélérateur de la cicatrisation par leur activité paracrine. Egalement, leurs propriétés immunosuppressives se traduiront localement par une diminution de l'inflammation et donc une amélioration de la qualité et de l'aspect esthétique de la cicatrice (qui dépend du degré d'inflammation).

### II.4. IDO est responsable de l'effet inhibiteur des cellules foetales sur la prolifération lymphocytaire

Les résultats sont présentés dans les graphiques a, b et c de la **figure 5B**.

Ces résultats mettent en évidence que les cellules foetales de l'invention (fibroblastes, kératinocytes ou fibroblastes + kératinocytes) inhibent bien la prolifération lymphocytaire, en l'absence de 1-MT. Dans tous les cas (fibroblastes, kératinocytes ou fibroblastes + kératinocytes), l'ajout de 1-MT à 200 µM lève l'inhibition de la prolifération de sorte qu'en présence de l'inhibiteur à cette concentration, la prolifération lymphocytaire revient pratiquement au même niveau que celle du témoin positif (lymphocytes + PHA-L + IL-2). De plus, on peut observer que l'effet du 1-MT est dose dépendant.

Ces résultats démontrent que l'inhibition de la prolifération lymphocytaire par les cellules fibroblastiques et kératinocytaires foetales de l'invention repose sur l'activité enzymatique d'IDO, ce qui est bien connu pour ce type de cellules.

### II.5. Evolution de la fermeture du scratch au cours du temps

Les résultats sont présentés sur le graphique de la **figure 6**.

Ces résultats mettent en évidence :
1- Une fermeture du scratch très rapide dès le début dans la condition Fibroblastes 50% / kératinocytes 50%. Ensuite stabilisation jusqu'à 15h puis de nouveau forte accélération de la couverture de la zone du scratch.
2- Dans la condition 100 % fibroblastes, la fermeture initiale est très lente puis augmente après 5 h mais la fermeture du scratch reste relativement modérée pour n'atteindre que 17 % à 20h.
3- Pour les conditions 100 % kératinocytes et 75 % fibroblastes / 25% kératinocytes la fermeture du scratch s'effectue de façon linéaire, depuis le début jusqu'à 20 heures pour atteindre environ 35 % de fermeture à la fin de l'acquisition.

### II.6. Fermeture du scratch au temps 5h et 20h en fonction des conditions.

Les résultats sont présentés sur les **figures 7A et 7B**. Cette autre représentation des résultats met bien en évidence l'importance du rapport kératinocytes / fibroblastes entre les différentes conditions au temps le plus précoce et à la fin de l'acquisition.

Par ce test de cicatrisation *in vitro* il apparaît que l'association de fibroblastes et de kératinocytes à un ratio bien précis de 50 %/50% permet une fermeture plus rapide de la zone de scratch par rapport à l'utilisation de fibroblastes ou de kératinocytes seuls. Ce ratio paraît particulièrement efficace dans les premiers temps de la fermeture de la zone de scratch.

### III- Exemples de substituts cutanés selon l'invention

### 111.1 Préparation d'un substitut cutané à base de Tissucol selon l'invention

Des cellules fibroblastiques et kératinocytaires foetales conservées dans de l'azote liquide suivant le protocole décrit ci-dessus ont été décongelées, ensemencées en flacon de culture 75 cm² et incubées à 37 °C en atmosphère humide avec 5% de CO₂ pendant 48 heures. Les cellules fibroblastiques et kératinocytaires ont ensuite été traitées séparément à la trypsine/EDTA 1X jusqu'au décollement complet du flacon de culture. Du milieu DMEM complémenté avec 10 % de SVF a alors été ajouté pour inhiber l'action de la tryspsine. Les cellules kératinocytaires et fibroblastiques ont ensuite été centrifugées puis comptées (toujours séparément). Un nombre égal de cellules fibroblastiques et de kératinocytaires (ratio kératinocytes : fibroblastes = 1 :1), de 0.2.10⁶ cellules à 10 x 10⁶, de préférence 2.10⁶, a alors été mélangé dans un même tube 15 ml. Ce tube a été centrifugé et le culot a été repris dans un volume de 1 mL de la deuxième composante du Tissucol kit (thrombine humaine + chlorure de calcium). La première composante du Tissucol® (fibrinogène humain + facteur III de coagulation humain + fibronectine humaine + plasminogène humain + aprotinine bovine) a alors été mélangée à volume égal avec la deuxième composante de la colle biologique grâce à l'utilisation du kit Tissucol 1 mL. Les deux composantes de la colle ainsi mélangées peuvent alors être déposées sur une plaie, une brûlure ou un ulcère.

### III.2 Préparation d'un substitut cutané à base de collagène I bovin selon l'invention

**III.2.a)** Des cellules fibroblastiques et kératinocytaires foetales conservées dans de l'azote liquide suivant le protocole décrit ci-dessus ont été décongelées, ensemencées en flacon de culture 75 cm² et incubées à 37 °C en atmosphère humide avec 5% de CO₂ pendant 48 heures. Les cellules fibroblastiques et kératinocytaires ont ensuite été traitées séparément à la trypsine/EDTA 1X jusqu'au décollement complet du flacon de culture. Du milieu DMEM complémenté avec 10 % de SVF a alors été ajouté pour inhiber l'action de la trypsine. Les cellules kératinocytaires et fibroblastiques ont ensuite été centrifugées puis comptées (toujours séparément). Un nombre égal de cellules fibroblastiques et kératinocytaires (ratio kératinocytes : fibroblastes = 1 :1) a été ensuite transféré dans un tube 15 ml, centrifugé et repris dans du milieu de culture DMEM complémenté avec 10 % de SVF. Les cellules fibrolastiques et kératinocytaires mélangées au ratio 1 :1 ont alors été ensemencées sur une matrice spongieuse de collagène bovin de type I à la densité de 0,1 à 5 x 10⁶ cellules par cm³ de matrice. Les cellules ensemencées sur la matrice ont été incubées à 37 °C en atmosphère humide avec 5 % de CO₂. Après 48 heures, la matrice cellularisée peut être appliquée sur la plaie, brûlure ou ulcère.
**III.2.b)** Le protocole décrit au point III.2.a) ci-dessus a été par la suite optimisé de la manière suivante : des cellules fibroblastiques et kératinocytaires foetales conservées dans de l'azote liquide ont été décongelées, puis directement ensemencées sur la matrice de collagène I bovin dès leur décongélation, sans passer par une remise en culture conformément au protocole III.2.a). Le ratio de 1:1 de kératinocytes : fibroblastes a également été maintenu. Les cellules ensemencées sur la matrice ont été ensuite incubées à 37 °C en atmosphère humide avec 5% de CO₂. Après 72h heures, la matrice cellularisée peut être appliquée sur la plaie, brûlure ou ulcère.

Ce protocole présente plusieurs avantages par rapport à celui exposé au point III.2.a) ci-dessus, puisqu'il permet :
(i) de limiter le nombre d'intervention des opérateurs, et ainsi de simplifier la préparation du substitut cutané ; et
(ii) de gagner 24h sur le temps de préparation du substitut cutané ce qui le rend plus rapidement disponible pour le patient.

Des dosages enzymatiques de l'activité LDH (lactate déshydrogénase) libérées par les cellules dans le substitut cutané ont par ailleurs démontré que ce nouveau mode de préparation permet une excellente survie des cellules. En particulier, ce procédé de préparation optimisé permet d'obtenir 85 % de viabilité cellulaire dans le pansement le jour de l'ensemencement, et 82 % 3 jours après l'ensemencement, en fin de préparation du substitut cutané.

### Références bibliographiques

Begaud B. Epidémiologie des ulcères de jambe. Ann Dermatol Venereol 2002 : 129 : 1225-1226
Bullard KM, Longaker MT, Lorenz HP. Fetal wound healing: current biology. World J Surg 2003: 27 : 54-61
Chester DL, Balderson DS, Papini RPG. A review of keratinocyte delivery to the wound bed. J Burn Care Rehabil 2004; 25: 266-275
Clark RAF, Lanigan JM, Della Pelle P, Manseau E, Dvorak HF, Colvin RB. Fibronectin and fibrin provide a provisional matrix for epidermal cell migration during wound reepithelialization. J Invest Dermatol 1982: 79: 264-269
Ho C, Tran K, Hux M, Campbell K. Greffes de peau artificielle dans le traitement des plaies chroniques: une méta-analyse de l'efficacité clinique et une étude coûts-efficacité. Rapport technologique n°52. Ottawa : Office canadien de coordination de l'évaluation des technologies de la santé 2005
Hohlfeld J, de Buys Roessingh A, Hirt-Burri et al. Tissue engineered fetal skin constructs for paediatric burns. Lancet 2005: 366: 840-842
Hunyadi J, Farkas B, Bertényi C, Olah J, Dobozy A. Keratinocyte grafting: a new means of transplantation for full-thickness wounds. J Dermatol Surg Oncol 1988: 14(1): 75-78
Jones JE, Nelson EA. Skin grafting for venous leg ulcers (review). The Cochrane library 2006: Issue 1
Rheinwald et Green. Serial cultivation of strains of human epidermal keratinocytes: the formation of keratinizing colonies from single cell. Cell 1975: 6: 331-344
Shen JT, Falanga V. Innovative therapies in wound healing. J Cutan Med Surg 2003: 217-224

## Revendications

1. Composition comprenant un mélange de cellules kératinocytaires humaines foetales et de cellules fibroblastiques humaines foetales, le ratio entre lesdites cellules kératinocytaires et fibroblastiques allant de 0,75 à 2,5.

2. Composition selon la revendication 1, dans laquelle ledit ratio est de 1 :1 ou de 7 :3.

3. Composition selon la revendication 1 ou 2, dans laquelle ledit mélange de cellules est intégré à une matrice extracellulaire non-immunogène.

4. Composition selon la revendication 3, **caractérisée en ce qu'**elle contient entre 0.1 et 5.10⁶ cellules par mL ou cm³ de matrice.

5. Composition selon la revendication 3 ou 4, **caractérisée en ce que** ladite matrice contient au moins un composé choisi dans le groupe consistant en : le collagène, la fibronectine, la fibrine, et l'élastine, et leurs mélanges.

6. Composition selon la revendication 3 ou 4, dans laquelle ladite matrice est composée de fibrinogène humain, de facteur XIII humain, de fibronectine humaine, de plasminogène, d'aprotinine bovine et de thrombine humaine.

7. Pansement contenant la composition telle que définie dans l'une quelconque des revendications 1 à 6.

8. Pansement selon la revendication 7, **caractérisée en ce qu'**il est stérile et emballé dans un contenant imperméable aux microorganismes.

9. Composition telle que définie selon l'une quelconque des revendications 1 à 6, pour son utilisation en tant que médicament.

10. Composition telle que définie selon l'une quelconque des revendications 1 à 6, pour son utilisation dans le traitement de tout déficit cutané, par exemple d'une plaie, d'une brûlure ou d'un ulcère.

11. Procédé de fabrication d'une composition telle que définie dans les revendications 1 à 6, comprenant l'étape de mélanger des cellules kératinocytaires humaines foetales et des cellules fibroblastiques humaines foetales à une matrice extracellulaire non-immunogène, dans un ratio allant de 0,75 à 2,5.

12. Procédé de fabrication selon la revendication 11, **caractérisé en ce que** ledit ratio est de 1 :1 ou de 7 :3.

13. Procédé de fabrication selon la revendication 11 ou 12, **caractérisé en ce qu'**il comprend, préalablement à ladite étape de mélange, les deux étapes suivantes:
a) obtenir des cellules kératinocytaires humaines foetales, et
b) obtenir des cellules fibroblastiques humaines foetales.

14. Procédé de fabrication selon la revendication 11 ou 12, **caractérisé en ce qu'**il comprend, au préalable de l'étape de mélange, les deux étapes suivantes:
a) obtenir une banque de cellules kératinocytaires humaines foetales, et
b) obtenir une banque de cellules fibroblastiques humaines foetales.

## Patentansprüche

1. Zusammensetzung, welche eine Mischung aus humanen fetalen keratinozytären Zellen und humanen fetalen fibroblastischen Zellen umfasst, wobei das Verhältnis zwischen den besagten keratinozytären und fibroblastischen Zellen im Bereich von 0,75 bis 2,5 liegt.

2. Zusammensetzung nach Anspruch 1, in welcher das besagte Verhältnis 1:1 oder 7:3 beträgt.

3. Zusammensetzung nach Anspruch 1 oder 2, in welcher die besagte Zellmischung in eine nicht immunogene extrazelluläre Matrix integriert ist.

4. Zusammensetzung nach Anspruch 3, **dadurch gekennzeichnet, dass** sie zwischen 0.1 und 5.10⁶ Zellen pro mL oder cm³ der Matrix enthält.

5. Zusammensetzung nach Anspruch 3 oder 4, **dadurch gekennzeichnet, dass** die besagte Matrix mindestens eine Verbindung enthält, welche ausgewählt ist aus der Gruppe bestehend aus: Kollagen, Fibronektin, Fibrin, Elastin, und deren Mischungen.

6. Zusammensetzung nach Anspruch 3 oder 4, in welcher die besagte Matrix aus humanem Fibrinogen, humanem Faktor XIII, humanem Fibronektin, Plasminogen, bovinem Aprotinin und humanem Thrombin besteht.

7. Wundauflage, welche die Zusammensetzung wie in einem der Ansprüche 1 bis 6 definiert enthält.

8. Wundauflage nach Anspruch 7, **dadurch gekennzeichnet, dass** sie steril und in einem für Mikroorganismen undurchlässigen Behältnis verpackt ist.

9. Zusammensetzung wie nach einem der Ansprüche 1 bis 6 definiert, für Verwendung als Heilmittel.

10. Zusammensetzung wie nach einem der Ansprüche 1 bis 6 definiert, für Verwendung in der Behandlung jeglichen Hautdefizits, zum Beispiel einer Wunde, einer Verbrennung oder eines Ulkus.

11. Verfahren zur Herstellung einer Zusammensetzung wie in den Ansprüchen 1 bis 6 definiert, welches den Schritt des Mischens der humanen fetalen keratinozytären Zellen und der humanen fetalen fibroblastischen Zellen mit einer nicht immunogenen extrazellulären Matrix in einem Verhältnis im Bereich von 0,75 bis 2,5 umfasst.

12. Herstellungsverfahren nach Anspruch 11, **dadurch gekennzeichnet, dass** das besagte Verhältnis 1:1 oder 7:3 beträgt.

13. Herstellungsverfahren nach Anspruch 11 oder 12, **dadurch gekennzeichnet, dass** es vor dem besagten Schritt des Mischens die folgenden zwei Schritte umfasst:
a) Gewinnen der humanen fetalen keratinozytären Zellen, und
b) Gewinnen der humanen fetalen fibroblastischen Zellen.

14. Herstellungsverfahren nach Anspruch 11 oder 12, **dadurch gekennzeichnet, dass** es vor dem Schritt des Mischens die folgenden zwei Schritte umfasst:
a) Gewinnen einer Bank an humanen fetalen keratinozytären Zellen, und
b) Gewinnen einer Bank an humanen fetalen fibroblastischen Zellen.

## Claims

1. Composition comprising a mixture of human foetal keratinocyte cells and human foetal fibroblast cells, the ratio between said keratinocyte and fibroblast cells ranging from 0.75 to 2.5.

2. Composition according to claim 1, wherein said ratio is 1:1 or 7:3.

3. Composition according to claim 1 or 2, wherein said mixture of cells is integrated into a non-immunogenic extracellular matrix.

4. Composition according to claim 3, **characterised in that** it contains between 0.1 and 5.10⁶ cells per mL or cm³ of matrix.

5. Composition according to claim 3 or 4, **characterised in that** said matrix contains at least one compound chosen from the group comprising: collagen, fibronectin, fibrin, and elastin, and mixtures thereof.

6. Composition according to claim 3 or 4, wherein said matrix is comprised of human fibrinogen, of human factor XIII, human fibronectin, plasminogen, bovine aprotinine and human thrombin.

7. Bandage containing the composition as defined in any of claims 1 to 6.

8. Bandage according to claim 7, **characterised in that** it is sterile and packaged in a container impermeable to microorganisms.

9. Composition as defined according to any of claims 1 to 6, for use as a drug.

10. Composition as defined according to any of claims 1 to 6, for use in the treatment of a skin defect, for example a wound, a burn or an ulcer.

11. Method for manufacturing a composition as defined in claims 1 to 6, comprising the step of mixing human foetal keratinocyte cells and human foetal fibroblast cells with a non-immunogenic extracellular matrix, in a ratio ranging from 0.75 to 2.5.

12. Method for manufacturing according to claim 11, **characterised in that** said ratio is 1:1 or 7:3.

13. Method for manufacturing according to claim 11 or 12, **characterised in that** it comprises, prior to said step of mixing, the following two steps:
a) obtaining human foetal keratinocyte cells, and
b) obtaining human foetal fibroblast cells.

14. Method for manufacturing according to claim 11 or 12, **characterised in that** it comprises, prior to the step of mixing, the following two steps:
a) obtaining a bank of human foetal keratinocyte cells, and
b) obtaining a bank of human foetal fibroblast cells.
